(19) Europäisches Patentamt
European Patent Office
Office européen des brevets

(11) Publication number: **0 218 474**
**A2**

(12) **EUROPEAN PATENT APPLICATION**

(21) Application number: **86307599.0**

(22) Date of filing: **02.10.86**

(51) Int. Cl.4: **C 12 N 15/00**
C 12 P 21/02

(30) Priority: **03.10.85 JP 221518/85**
**02.09.86 JP 205036/86**

(43) Date of publication of application:
**15.04.87 Bulletin 87/16**

(84) Designated Contracting States:
**AT BE CH DE ES FR GB GR IT LI LU NL SE**

(71) Applicant: **Takeda Chemical Industries, Ltd.**
**27, Doshomachi 2-chome Higashi-ku**
**Osaka-shi Osaka, 541 (JP)**

(72) Inventor: **Onda, Haruo**
**21-6, Aza-Junmatsu Tada-in**
**KAWANISHI Hyogo 666-01 (JP)**

**Inada, Takashi**
**14-12, Minami-gyotoku 3-chome**
**Ichikawa Chiba 272-01 (JP)**

**Igarashi, Koichi**
**66-3, Shimogamo Miyazaki-cho**
**Sakyo-ku, Kyoto 606 (KP)**

(74) Representative: **Laredo, Jack Joseph et al**
**Elkington and Fife High Holborn House 52/54 High Holborn**
**London, WC1V 6SH (GB)**

The applicant has filed a statement in accordance with Rule 28 (4) EPC (issue of a sample only to an expert).
Claims for the following Contracting State: AT.

(54) **Novel DNA and polypeptide.**

(57) This invention relates to a polypeptide exhibiting hepatitis B virus e antigenicity produced by a microorganism; a recombinant DNA comprising a DNA fragment coding for a polypeptide exhibiting hepatitis B virus e antigenicity; a recombinant DNA comprising a DNA fragment coding for a polypeptide exhibiting hepatitis B virus c antigenicity, wherein the nucleotide sequence between the SD sequence of the promoter and the translational initiation codon is ATCGGGC; and a method of preparing polypeptides exhibiting hepatitis B virus e and c antigenicity respectively, which comprises cultivating transformants containing above-described recombinant DNAs respectively.

The thus obtained polypeptide exhibiting HBc or HBe antigenicity may be used as a diagnostic reagent for HBV infection and a defensive (preventing) agent against HBV infection.

A recombinant DNA having a base sequence of ATCGGGC between the SD sequence of its promoter region and the translational initiation codon affords a gene product having HBc or HBe antigenicity with a high yield and is advantageously used for the production of HBc or HBe antigen.

EP 0 218 474 A2

**Description**

NOVEL DNA AND POLYPEPTIDE

Background of the Invention

This invention relates to a novel DNA and to a polypeptide.

Human hepatitis B occurs upon infection with hepatitis B virus (hereinafter referred to as HBV) which is a sort of a DNA virus. HBV is known as "Dane particle". There exists HBV surface antigen (hereinafter referred to as HBsAg) at the surface of the virus particle. Within the particle, are present circular double-stranded DNA with a single-stranded portion, HBV core antigen (hereinafter referred to as HBcAg) and HBV e antigen (hereinafter referred to as HBeAg). Also, an activity of endo-geneous DNA synthetase which serves to repair the single-stranded portion of the double-stranded DNA is detected in the particle. HBV DNA has a molecular weight of $2.1 \times 10^6$ daltons and is composed of about 3200 base pairs. Four types of antigenicity of HBsAg are known, i.e. adr, adw, ayr and ayw, with the common antigen "a" as their center.

HBcAg may be used as a diagnostic reagent for early stage diagnosis of persons infected with HBV. That is, when a person is infected with HBV, anti-HBc antibody is induced in the blood before the occurrence of HBs antibody. Thus, whether or not a person is infected with HBV can be determined at an early stage of infection by detecting the anti-HBc antibody in serum. HBcAg is recently reported as being effective for the prevention of HBV infection.

The blood containing HBeAg also contains Dane particles in a large amount and has a danger of causing HBV infection. Thus, to detect the presence of HBeAg in blood is important in curing HBV infected patients and is essential for diagnosis of HBV infection.

In order to obtain HBcAg and HBeAg, it is necessary to collect Dane particles and to isolate HBcAg and HBeAg therefrom. Since the amount of HBcAg and HBeAg contained in the Dane particles is very small, there has been a great demand for a method which can produce HBcAg and HBeAg in a large amount and in a safe manner.

Summary of the Invention

In accordance with the present invention there is provided (I) a polypeptide which exhibits hepatitis B virus e antigenicity and which is produced by a microorganism, and (2) a recombinant DNA comprising a promoter, a translational initiation codon located downstream from the promoter, a DNA fragment which codes for a polypeptide exhibiting hepatitis B virus e antigenicity and which is located downstream from the translational initiation codon, and a stop codon joined immediately after the DNA fragment, (3) a recombinant DNA comprising a promoter, a translational initiation codon located downstream from the promoter, a DNA fragment which codes for a polypeptide exhibiting hepatitis B virus c antigenicity and which is located downstream from the promoter, and a stop codon joined immediately after the DNA fragment, wherein the nucleotide sequence between the SD sequence of the promoter and the translational initiation codon is ATCGGGC, and (4) a method of preparing a polypeptide exhibiting hepatitis B virus c antigenicity, which comprises cultivating a transformant containing a recombinant DNA comprising a promoter, a translational initiation codon located downstream from the promoter, a DNA fragment which codes for a polypeptide exhibiting hepatitis B virus c antigenicity and which is located downstream from the promoter, and a stop codon joined immediately after the DNA fragment, wherein the nucleotide sequence between the SD sequence of the promoter and the translational initiation codon is ATCGGGC.

HBc and HBe antigens are obtainable naturally only from humans or chimpanzees infected with HBV in only a limited amount. The handling of such infected bodies encounters a danger of HBV infection. The present invention has solved these problems and has enabled the production of a large amount of a polypeptide which exhibits HBc or HBe antigenicity similar to the natural antigen and which is safe and inexpensive.

Brief Description of the Drawings

Fig. I shows the entire nucleotide sequence of adw-type HBV DNA;

Fig. 2 shows the amino acid sequence of adw-type HBcAg;

Fig. 3 shows the nucleotide sequence of a DNA coding for adw-type HBcAg;

Fig. 4 is a scheme for constructing the plasmid pTB368;

Fig. 5 is a scheme for constructing the expression plasmid for HBeAg;

Fig. 6 shows the results of Western blotting (lanes I and 2) of the product of Escherichia coli DHI/pTB368;

Fig. 7 shows the results of analysis of the product of Escherichia coli DHI/pTB368 by cesium chloride equilibrium density gradient centrifugation;

Figs. 8 and 9 show the results of analysis of the products of Escherichia coli DHI/pTB368 and DHI/pTB44I by sucrose gradient centrifugation; and

Fig. I0 shows the test results of neutralization with rabbit anti-HBc antibody using HBe(HBc) RIA.

Fig. II is a scheme for constructing the plasmid pTB(4) 369-4.

Fig. I2 is a scheme for constructing the plasmid pTB(4) 369-32 and pTB(4) 369-4I.

Fig. I3 is a scheme for constructing the plasmid pTB(4) 44I-I.

Fig. 14 is a scheme for constructing the plasmid pTB(4) 441-8.

Fig. 15 is a scheme for constructing the plasmid pTB(4) 552-8.

Fig. 16 and Fig. 17 show the results of analysis of the molecular weight of the product of Escherichia coli DHI/pTB(4) 441-I and Escherichia coli DHI/pTB(4) 552-8. In Fig 16 the Western blotting method using $^{125}$I-anti-HBe antiserum is employed and in Fig. 17 an immunoprecipitation method using human anti-HBe antiserum.

## Detailed Description of the Invention

Any microorganism may be used for the purpose of the present invention as long as it can produce a polypeptide showing hepatitis B virus e antigenicity. Examples of suitable microorganisms include Escherichia coli and yeasts containing a recombinant DNA bearing a promoter, a translational initiation codon located downstream from the promoter, a DNA fragment which codes for a polypeptide exhibiting hepatitis B virus e antigenicity and which is located downstream from the translational initiation codon, and a stop codon joined immediately after the DNA fragment. Escherichia coli containing the above-mentioned recombinant DNA is especially preferred.

Any promoter containing a region or site to which RNA polymerase is able to bind for the initiation of mRNA synthesis may be used for the purpose of the present invention. Illustrative of promoters suitably used when Escherichia coli is employed as a host are trp promoter, recA promoter, lac promoter, $\lambda P_L$ promoter and tufB promoter. Above all the use of trp promoter is especially preferred.

A promoter providing a nucleotide sequence of ATCGGGC between its SD (Shine-Dalgarno) sequence and a translational initiation codon which will be described hereinafter is more preferably used.

When a yeast is used as a host, any promoter may be used as long as it can function in the host.

As the translational initiation codon, ATG may be preferably used.

As the DNA coding for a polypeptide exhibiting hepatitis B virus c antigenicity, there may be used DNA coding for a polypeptide exhibiting hepatitis B virus c antigenicity of any subtype. It is preferable, however, to use a DNA coding for the polypeptide shown in Fig. 2 (adw type). The use of the DNA between 1901 and 2455 in the nucleotide sequence shown in Fig. I (adw type) is more preferable.

As the DNA coding for a polypeptide exhibiting hepatitis B virus e antigenicity, there may be used a DNA coding for a polypeptide exhibiting hepatitis B virus e antigenicity of any subtype. It is preferable, however, to use a DNA coding for the polypeptide between I and 95 to 184 in the amino acid sequence shown in Fig. 2. The use of the DNA between 1901 and 2263 to 2356 (adw type) is more preferred. (The base at the position 2263 can be C.)

The DNA coding for a polypeptide showing hepatitis B virus c or e antigenicity may be a DNA coding for a polypeptide in which a portion of its amino acid or peptide is deleted or replaced with another amino acid or peptide or in which an amino acid or a peptide is added, as long as the polypeptide produced by the DNA can exhibit desired antigenicity.

As the stop codon, there may be mentioned TAA, TAG or TGA.

A DNA coding for a polypeptide showing adw-type HBc antigenicity may be prepared in a manner as follows. Plasmid pBR322-EcoRI/HBV933 (hereinafter referred to as pHBV933) disclosed in Nucleic Acids Res. II, 1747 (1983) is digested with restriction enzyme HhaI to obtain DNA fragments. From the DNA fragments, a DNA of about IKb containing a region coding for a polypeptide showing HBc antigenicity is isolated, ligated with an EcoRI linker and inserted into the EcoRI site of trp promoter-containing plasmid ptrp 781 [plasmid prepared by digesting ptrp 701 (European Laid Open Patent Publication No. 0068719) with restriction enzyme EcoRI, followed by partial digestion with ClaI, treatment with DNA polymerase I large fragment for repairing the cohesive ends and circularization with T4 DNA ligase; The ClaI site located near the EcoRI site is destroyed] to obtain plasmid pHEI. This plasmid is digested with EcoRI and then with nuclease Bal 31, ligated with a ClaI linker and inserted into the ClaI site of plasmid ptrp 771 bearing trp promoter (European Laid Open Patent Publication No. 0068719), thereby to obtain plasmid pTB368 containing the desired DNA coding for a polypeptide exhibiting HBc antigenicity(Fig. 4). By transforming Escherichia coli (such as a strain DHI, τ 1776 or C600) with plasmid pTB368, there is obtained a transformed strain of Escherichia coli capable of producing a polypeptide showing HBc antigenicity.

DNA coding for a polypeptide showing adw type HBe antigenicity may be prepared, for example, by digesting plasmid pTB368 with a suitable restriction enzyme such as AvaI or AvaII and then, if necessary, with nuclease Bal31, ligating the resulting fragment with a suitable linker containing a stop codon and inserting the resultant DNA into plasmid ptrp 771. By transforming Escherichia coli (such as a strain DHI, τ1776 or C600) with the thus obtained plasmid, there is obtained a transformed strain of Escherichia coli capable of producing a polypeptide showing HBe antigenicity(Fig. 5).

A DNA coding for a polypeptide exhibiting HBc or HBe antigenicity of a subtype other than adw may be obtained in the same manner as above. When a microorganism other than Escherichia coli is used as a host, transformants capable of producing the desired polypeptide may be obtained by inserting the above DNA into a plasmid bearing a promoter capable of functioning in the host.

A recombinant DNA having a nucleotide sequence of ATCGGGC between the SD sequence of its promoter and the translational initiation codon may be obtained through a series of treatments including, for example, treatment with a suitable restriction enzyme such as EcoRI, treatment with SI nuclease and ligation.

Alternatively, such a recombinant DNA may be prepared by oligonucleotide mutagenesis or any other suitable known method.

The thus obtained transformant of, for example, Escherichia coli may be cultivated in any known medium such as L broth, Penassay broth or M-9 medium containing glucose and casamino acids. The culture medium may be supplemented with a suitable additive such as 3β-indolylacrylic acid for the purpose of improving the promoter activity. Cultivation is performed at a temperature of I5-43°C, preferably 28-40°C, for 2-24 hours, preferably 3-8 hours. Aeration and agitation may be conducted, as necessary.

After cultivation, cells are collected in any known manner, suspended in a buffer and disrupted by sonication, lysozyme and/or freezing-thawing. The disrupted mixture is centrifuged to obtain an extract containing the desired polypeptide. Isolation and purification of the polypeptide from the extract is conducted in conventional manner.

A transformant of a microorganism other than Escherichia coli may be treated in the same manner as above to recover the desired polypeptide.

The thus obtained polypeptide exhibiting HBc or HBe antigenicity may be used as a diagnostic reagent for HBV infection and a defensive (preventing) agent against HBV infection.

A recombinant DNA having a base sequence of ATCGGGC between the SD sequence of its promoter region and the translational initiation codon affords a polypeptide having HBc or HBe antigenicity with a high yield and is advantageously used for the production of HBc or HBe antigen.

The present invention will now be described in detail below by way of various examples. It is to be understood, however, that these examples are not to be considered as limiting the scope of the invention.

Example I

Plasmid pHBV933 was digested with restriction enzymes EcoRI and HhaI. The digest was electrophoresed on I% agarose gel to isolate a DNA fragment (I005 bp) bearing the HBc antigen gene. The DNA fragment was subjected to a nuclease SI treatment in a buffer (I0 mM sodium acetate, I50 mM NaCl, 0.05 mM $ZnSO_4$, pH 4.0), ligated with an EcoRI linker d(GGAATTCC) and then treated with EcoRI. The resultant DNA fragment was inserted into the EcoRI site of plasmid ptrp78I and introduced into Escherichia coli DHI. Plasmid pHBcHEI bearing the HBcAg gene was extracted from the clones and was cleaved with EcoRI. EcoRI DNA fragment (5μg) was treated with 2 units of Bal3I in a buffer (600 mM NaCl, 20 mM Tris•HCl, pH 8.0, I2 mM $CaCl_2$, I2mM $MgCl_2$, I.0 mM EDTA) at 24°C for I minute, ligated with a ClaI linker d(CATCGATG) and digested with ClaI. The thus treated DNA fragment was then introduced into Escherichia coli DHI for transformation, thereby to obtain tetracycline-resistant transformant Escherichia coli DHI/pTB368.

Example 2

The transformant obtained in Example I was cultivated at 37°C in M-9 medium (I0 ml) containing 8 μg/ml of tetracycline. When the Klett unit (absorbance at 580 nm) of the medium reached I80-200, 3β-indolylacrylic acid was added thereto to a concentration of 25 μg/ml and the cultivation was further continued for 2-3 hours.

The culture was centrifuged at 5000 rpm for I0 minutes for harvesting cells. The cells were then suspended in I ml of a buffer (20 mM Tris•HCl, pH 7.6, 20% sucrose), to which were added lysozyme (3 mg/ml), EDTA (final concentration of 5 mM) and PMSF (final concentration of ImM). The mixture was agitated sufficiently and allowed to stand at 4°C for I hour. The mixture was then subjected to sonication for further disrupting the cells and, thereafter, centrifuged twice at I5000 rpm for 20 minutes. The supernatant (extract) thus obtained was tested for HBc antigenic activity by HBe RIA kit(Abbott). As a result, the transformant was found to be an HBc antigen expression clone.

The cell extract from the transformant was diluted with isotonic sodium chloride solution for measuring HBc antigenic activity. As a result, a sample diluted with 2500 volumes of physiological saline solution still permitted the detection of its HBc antigenic activity.

Example 3

Plasmid pTB368 was isolated from the above-described transformant Escherichia coli DHI/pTB368, then cleaved with restriction enzyme AvaI and treated with nuclease Bal 3I for 30-90 sec. The resulting fragment was ligated with an EcoRI linker having a stop codon and inserted into the ClaI•EcoRI site of ptrp77I. The resultant plasmid was introduced into Escherichia coli DHI for transformation. The transformants were cultivated in M-9 medium and HBe antigenic activity was measured in the same manner as described in Example 2. Transformants Escherichia coli DHI/pTB449, Escherichia coli DHI/pTB44I and Escherichia coli DHI/pTB552 capable of producing HBe antigen were obtained.

Example 4

The cell extracts from the above transformants were each diluted to five volumes with physiological saline solution and assayed for antigenic activity by means of HBe RIA kit(Abbott). The results were as shown below.

| | cpm | P/N |
|---|---|---|
| Positive control | 4942 | |
| Negative control | 979 | |
| E. coli DH1/pTB368 | 49736 | 50.8 |
| E. coli DH1/pTB441 | 19003 | 19.4 |
| E. coli DH1/pTB449 | 11774 | 12.0 |
| E. coli DH1/pTB552 | 7034 | 7.1 |

$$P/N = \frac{\text{Positive cpm}}{\text{Negative control cpm}}$$

Example 5

(i) The cell extract from Escherichia coli DHI/pTB368 was centrifuged at 40000 rpm at 4°C for 6 hours using a Beckman ultracentrifuge (SW 55 rotor) and the precipitate was dissolved in a buffer (20 mM Tris•HCl, ImM EDTA, 0.I5 M NaCl, pH 7.6).The resulting sample (50 μl) was mixed with 50 μl of a buffer for SDS polyacrylamide (0.I5 M Tris•HCl, pH 6.8, 4% SDS, 20% glycerol, I0% 2-mercaptoethanol, 0.002% bromophenol blue). The mixture was heated at I00°C for 5-I0 minutes and electrophoresed on a polyacrylamide gel.The protein thus isolated was electrically transferred on a nitrocellulose filter and reacted with $^{125}$I-anti-HBe antiserum, followed by washing and autoradiography. As a result, the protein produced by the transformant was expected to have a molecular weight of 2I500-22000 daltons(Fig. 6).

(ii) Solid cesium chloride was added to the cell extract from Escherichia coli DHI/pTB368 so that the resulting mixture had average ρ of I.20. The mixture was then centrifuged at 38000 rpm for 72 hours for fractionation. The fractions were then assayed for HBc antigenicity. The HBc antigen was found to be fractionated as a peak at about ρ =I.34.

From the results obtained in (i) and (ii) above, the expression product by the transformant is expected to constitute particles of HBcAg.

(iii) Each of the cell extracts (0.5 ml) from Escherichia coli DHI/pTB368 and DHI/pTB44I was laid over 35 ml of 5-25% sucrose gradient solution (20 mM Tris, PH 7.6, 0.I5M NaCl, 0.00I M EDTA) and centrifuged at 24000 rpm at 4°C for 4 hours and divided into 30 fractions. 200 μl of each of the fractions was assayed for HBcAg and HBeAg by means of a commercial HBeAg EIA (enzyme immunoassay) kit(Abbott). The gene product from Escherichia coli DHI/pTB368 was found mainly in the fractions 9 and I0, while the product from Escherichia coli DHI/pTB44I was found in the fractions 20 through 23(Fig. 8). The former product is expected to be a polypeptide in the form of a particle, while the latter is expected to be a polypeptide which does not form a particle.

EXAMPLE 6

The cell extract from Escherichia coli DHI/pTB368 was mixed with Anti-HBc antiserum obtained by immunizing with HBcAg particles extracted from liver of a chimpanzee infected with HBV for measuring the reduction of antibody titre by means of a commercial anti-HBc assay kit (Abbott). As a result, the extract is found to react with and neutralize the anti-HBc antibody. In contrast, it was found that the cell extracts from Escherichia coli DHI/pTB44I, DHI/pTB449 and DHI/pTB552 hardly reacted with the anti-HBc antibody.

The extract from each transformant was reacted with $^{125}$I-anti-HBc antibody. The remaining amount of the $^{125}$I-anti-HBc antibody was then measured with the use of anti-HBc antibody assay kit (Abbott) to obtain the results shown below.

| | Inhibition | |
|---|---|---|
| | cpm | % |
| Positive control | 324 | 100 |
| Negative control | 12013 | 0 |
| E. coli DH1/pTB368 | 3601 | 72 |
| E. coli DH1/pTB441 | 12920 | -8.3 |
| E. coli DH1/pTB449 | 11905 | 0.9 |
| E. coli DH1/pTB552 | 12832 | -7.0 |

Example 7

The cell extracts from Escherichia coli DHI/pTB368 and DHI/pTB44I were each diluted with physiological saline solution to 64 volumes and 4 volumes, respectively so that each diluted sample could show an antigenic activity count of about 20000 cpm. The diluted samples and undiluted serum HBeAg (each I00 µl) were each added with a solid phase HBe (HBc) antibody along with rabbit anti-HBc antibody (diluted with isotonic sodium chloride solution to I00, 500 and I000 volumes). As a control, the isotonic sodium chloride solution was used in place of the anti-HBc antibody liquid. Each mixture was allowed to react at room temperature overnight and, thereafter, the antigen bound to the solid phase was measured. The results are shown in Fig. I0 as percentages based on the counts of respective systems added with the control (physiological saline solution). The counts in the control systems were 22340 cpm, 2347I cpm and 949I cpm for DHI/pTB368, DHI/pTB44I and serum HBeAg, respectively.

In the case of the serum HBeAg, the reduction of the count relative to the control was only I0% at the maximum even though the amount of the antigen added was about a half of those in the diluted extract samples. This suggests that the anti-HBc antibody has little influence upon the detection of HBeAg.

The count of the extract from Escherichia coli DHI/ pTB368 decreases upon addition of the anti-HBc antibody, indicating that the expressed product has HBc antigenicity. Such a conspicuous decrease in count as seen in the extract from Escherichia coli DHI/pTB368 does not occur in the case of the extract from Escherichia coli DHI/pTB44I. This suggests that the gene product from the latter has HBe antigenicity.

Example 8

The base sequences of the 3′-terminal side of the DNAs coding for HBeAg, which are inserted into plasmids pTB44I, pTB449 and pTB552 are shown below:

(i) pTB44I

```
CCA · CCA · AAT · GCC · CCT · AGA · ATT ·
Pro   Pro   Asn   Ala   Pro   Arg   Ile
(134)

CTT · GAA · GAC · GAA · AGG · GCC · TCG · TGA
Leu   Glu   Asp   Glu   Arg   Ala   Ser    -
```

The peptide produced with the plasmid pTB44I lacks the 47 amino acids of the C-terminal side of HBcAg (lacks the 7 amino acids of the C-terminal side of natural HBeAg) and has other 9 amino acids replaced therefor.

(ii) pTB449

```
AGA · CGA · CGG · CTA · GAA · TTC · TTG ·
(2348)

AAG · ACG · AAA · GGG · CCT · CGT · GAT ·

ACG · CCT · ATT · TTT · ATA · GGT · TAA ·
```

The peptide produced with the plasmid pTB449 lacks the 33 amino acids of the C-terminal side of HBcAg

and has 16 amino acids replaced therefor.

(iii) pTB552 GTA • CTT • GAA • TAT • TTG • GTC • TCC • TAG (2243)

The peptide produced with the plasmid pTB552 lacks the 64 amino acids of the C-terminal side of HBcAg.

Example 9

Plasmid pTB368 was digested with EcoRI and treated with exonuclease Bal31. The resulting fragment was ligated with a PstI linker d(GCTGCAGC) and then treated with ClaI and PstI DNA, thereby to obtain a ClaI•PstI fragment bearing the HBcAg gene. This DNA fragment was inserted into the ClaI•PstI site of ptrp771. The resultant plasmid was digested with ClaI, ligated with an EcoRI linker d(GGAATTCC), treated with EcoRI and PstI and then inserted into the EcoRI site of ptrp781. The resulting plasmid was introduced into Escherichia coli DHI for transformation, thereby to obtain a transformant, Escherichia coli DHI/pTB(4)368-I.

The transformant was cultivated in M-9 medium and cells were extracted therefrom. The cell extract was assayed for HBcAg yield to reveal that the transformant produced about 10 times as much HBcAg as Escherichia coli DHI/pTB368.

The base sequence of a region between the SD sequence of the plasmid pTB(4)368-I and the translational initiation codon of its HBcAg gene was determined in conventional manner. The region was found to be composed of 14 bp as follows:

5′ AAAA GTATCGAAGGCCGGGCATG

For the purpose of decreasing the number of the nucleotides between the SD sequence and ATG, plasmid pTB(4)368-I was cleaved with EcoRI, treated with SI nuclease and then subjected to ligation reaction. Escherichia coli DHI was transformed with the reaction mixture to obtain a transformant DHI/pTB(4)369 containing plasmid pTB(4)369. The transformant was found to produce about 105 times as much HBcAg as Escherichia coli DHI/pTB368. The base sequence between the SD sequence and ATG of plasmid pTB(4)369 involves 7 bp as follows:

5′ AAAAGGGTATCGGGCATG

Example 10

(i) Plasmid pTB(4)369 was digested with AvaI and repaired with Klenow fragment to render the single-stranded portions to double stranded, followed by treatment with PstI and isolation of a large fragment. The large fragment was then ligated with a PstI stop linker;

5′ ATAACTAACTAACTGCA 3′

3′ TATTGATTGATTG 5′

having a recognition site for PstI, followed by treatment with PstI, isolation of a large fragment and ligation, to obtain plasmid pTB(4)369-4(Fig. 11).

(ii) Plasmid pTB(4)369 was digested with StyI. The obtained small fragment was treated with AvaII and repaired with Klenow fragment. The resulting double-stranded fragment was ligated with the above-described PstI stop linker, followed by treatment with HpaI and PstI and isolation of 502 bp fragment. This fragment was inserted into the HpaI•PstI site of ptrp781 to obtain plasmid pTB(4)369-32 (Fig.12).

(iii) Plasmid pTB(4)369 was digested with StyI. The small fragment thus obtained was treated with HpaII and repaired with Klenow fragment. The resulting double-stranded fragment was ligated with the above-described PstI stop linker, followed by treatment with HpaI and PstI and isolation of 472 bp fragment. This fragment was inserted into the HpaI•PstI site of ptrp771 to obtain plasmid pTB(4)369-41(Fig. 12).

(iv) Plasmid pTB441 was cleaved at one of the two BglII sites (5′ side one) of the HBeAg gene and at the PstI site of the vector region. The resulting DNA fragment was inserted at the BglII (5′ side one)•PstI site of pTB(4)369 to obtain plasmid pTB(4)441-1(Fig. 13).

(v) Plasmid pTB441 was cleaved at one of the two BglII sites (3′ side one) of the HBeAg gene and at the PstI site of the vector region. The resulting DNA fragment was inserted at the BglII (5′ side one)•PstI site of pTB(4)369 to obtain plasmid pTB(4)441-8(Fig. 14).

(vi) Plasmid pTB552 was cleaved at one of the two BglII sites (5′ side one) of the HBeAg gene and at the PstI site of the vector region. The resulting DNA fragment was inserted at the BglII (5′ side one)•PstI site of pTB(4)369 to obtain plasmid pTB(4)552-8.

Example 11

Transformants of Escherichia coli DHI containing the plasmids obtained in Examples 9 and 10 were cultivated in the same manner as that in Example 2. The cells were collected and disrupted, after the addition of EDTA and lysozyme, by sonication to obtain cell extracts. Each cell extract was tested for HBcAg and HBeAg with the use of HBe EIA (Abbott) and Corzyme. As a result, the transformant of Escherichia coli containing plasmid pTB(4)369, pTB(4)369-4, pTB(4)369-32 or pTB(4)369-41 was found to produce HBcAg and HBeAg, while the transformant containing plasmid pTB(4)441-I or pTB552-8 was found to produce HBeAg.

The extract from the transformant of Escherichia coli containing pTB(4)441-8 was diluted and assayed for antigenic activity using a commercial HBe RIA kit to obtain the results shown below.

| Dilution ratio (times) | pTB(4)441-8 $^{125}$I count (cpm) |
|---|---|
| 5 | 24915 |
| 10 | 25843 |
| 20 | 27071 |
| 40 | 29788 |
| 80 | 24811 |
| 160 | 16001 |
| 320 | 11395 |
| 640 | 8450 |
| 1280 | 5866 |
| 2560 | 4057 |

Negative control 500 cpm

Example l2

The 3'-terminal nucleotide sequences of the DNAs coding for HBeAg which are inserted into plasmids pTB(4) 369-4, pTB(4)369-32 and pTB(4)369-4l are shown below:

i) pTB(4)369-4

```
ATA · ACT · AAC · TAA
Ile   Thr   Asn   -
(182)
```

ii) pTB(4)369-32

```
ATA · ACT · AAC · TAA
Ile   Thr   Asn   -
(154)
```

iii) pTB(4)369-41

```
ATA · ACT · AAC · TAA
Ile   Thr   Asn   -
(145)
```

Plasmid pTB(4)44l-8 lacks both the region of the HBcAg gene coding for the peptide from the 29th to 82nd amino acids and the region coding for the C-terminal side 47 amino acids but has a newly incorporated region coding for 9 amino acids.

Example l3

The molecular weight of the HBeAg antigenicity-exhibiting polypeptide product of Excherichia coli DHl containing plasmid pTB(4)44l-l or pTB(4)552-8 was determined by the Western blotting method using

$^{125}$I-anti-HBe antiserum and by an immunoprecipitation method using human anti-HBe antiserum. The Western blotting method analysis[Towbin, H., Proc. Natl. Acad. Sci., U.S.A. 76, 9, 4350-4354(1979)] revealed that the molecular weight of the product of Escherichia coli containing pTB(4)441-l was 15000-15500 daltons. The immunoprecipitation method which was carried out by cultivating Escherichia coli DH-l containing pTB(4)552-8 in M-9 medium in the presence of $^{14}$C-amino acids, adding human anti-HBe antiserum to the cell extract, reacting the resulting mixture at 37°C, recovering the immunoreaction product by the addition of Protein A (10% solution), electrophoresing the product on polyacrylamide(17%) gel and conducting autoradiography, revealed that the gene product had a molecular weight of 13000-13500 daltons(Fig. 17).

The transformants are deposited at the depositories under the deposition accession numbers shown below;

| | IFO | FERM |
|---|---|---|
| E. coli DH1/pTB368 | 14467 | BP-1147 |
| E. coli DH1/pTB441 | 14468 | BP-1148 |
| E. coli DH1/pTB552 | 14469 | BP-1149 |
| E. coli C 600 | 14410 | BP-808 |
| E. coli DH1/pTB(4)369 | 14533 | BP-1153 |
| E. coli DH1/pTB(4)441-1 | 14534 | BP-1154 |
| E. coli DH1/pTB(4)441-8 | 14535 | BP-1155 |
| E. coli DH1/pTB(4)552-8 | 14536 | BP-1156 |

The numbers below "IFO" represent deposition accession numbers in the Institute for Fermentation, Osaka, Japan and those below "FERM" represent deposition accession numbers in Fermentation Research Institute, Agency of Industrial Science and Techonolgy, Ministry of International Trade and Industry, Japan.

Escherichia coli DHI is a known microorganism [M. E. Selson et al, Nature 217, 1110 (1968)].

Plasmid pTB449 is derived from pTB368 and plasmids pTB(4)369-4, pTB(4)369-32 and pTB(4)369-41 are derived from pTB(4)369, as mentioned in Examples 3 and 10. Transformants carrying the key plasmids pTB368 and pTB(4)369 have been deposited as shown above.

**Claims**

(1) A polypeptide which exhibits hepatitis B virus e antigenicity and which is produced by a microorganism.

(2) A recombinant DNA comprising a promoter, a translational initiation codon located downstream from the promoter, a DNA fragment which codes for a polypeptide exhibiting hepatitis B virus e antigenicity and which is located downstream from the translational initiation codon, and a stop codon joined immediately after the DNA fragment.

(3) A recombinant DNA according to claim 2, which is one of the following plasmids;
pTB 441 (FERM BP-1148)
pTB 449
pTB 552 (FERM BP-1149)
pTB(4)441-1 (FERM BP-1154)
pTB(4)441-8 (FERM BP-1155)
and
pTB(4)552-8 (FERM BP-1156)

(4) A method of preparing a polypeptide exhibiting hepatitis B virus e antigenicity, which comprises cultivating a transformant containing a recombinant DNA comprising a promoter, a translational initiation codon located downstream from the promoter, a DNA fragment which codes for a polypeptide exhibiting hepatitis B virus e antigenicity and which is located downstream from the promoter, and a stop codon joined immediately after the DNA fragment, wherein the nucleotide sequence between the SD sequence of the promoter and the translational initiation codon is ATCGGGC.

(5) A recombinant DNA comprising a promoter, a translational initiation codon located downstream from the promoter, a DNA fragment which codes for a polypeptide exhibiting hepatitis B virus c antigenicity and which is located downstream from the promoter, and a stop codon joined immediately after the DNA fragment, wherein the nucleotide sequence between the SD sequence of the promoter and the translational initiation codon is ATCGGGC.

(6) A recombinant DNA according to claim 5 which is one of the following plasmids;
pTB(4)369 (FERM BP-II53)
pTB(4)369-4
pTB(4)369-32
and
pTB(4)369-4I

(7) A method of preparing a polypeptide exhibiting hepatitis B virus c antigenicity, which comprises cultivating a transformant containing a recombinant DNA comprising a promoter, a translational initiation codon located downstream from the promoter, a DNA fragment which codes for a polypeptide exhibiting hepatitis B virus c antigenicity and which is located downstream from the promoter, and a stop codon joined immediately after the DNA fragment, wherein the nucleotide sequence between the SD sequence of the promoter and the translational initiation codon is ATCGGGC.

(8) A polypeptide which exhibits hepatitis B virus c and e antigenicity and which is produced by a microorganism.

(9) A polypeptide which exhibits hepatitis B virus c or e antigenicity and which is produced by a microorganism.

Claims for the following Contracting State: AT

I. A method of preparing a polypeptide exhibiting hepatitis B virus e antigenicity which comprises cultivating a transformant containing a recombinant DNA comprising a promoter, a translational initiation codon located downstream from the promoter a DNA fragment which codes for a polypeptide exhibiting hepatitis B virus e antigenicity and which is located downstream from the translational initiation codon, and a stop codon joined immediately after the DNA fragment.

2. A method according to Claim I, in which the recombinant DNA is one of the following plasmids:
pTB 44I (FERM BP-II48)
pTB 449
pTB 552 (FERM BP-II49)
pTB(4)44I-I (FERM BP-II54)
pTB(4)44I-8 (FERM BP-II55)
and
pTB(4)552-8 (FERM BP-II56).

3. A method of preparing a polypeptide exhibiting hepatitis B virus e antigenicity which comprises cultivating a transformant containing a recombinant DNA comprising a promoter, a translational initiation codon located downsteam from the promoter, a DNA fragment which codes for a polypeptide exhibiting hepatitis B virus e antigenicity and which is located downstream from the promoter, and a stop codon joined immediately after the DNA fragment wherein the nucleotide sequence between the SD sequence of the promoter and the translational initiation codon is ATCGGGC.

4. A polypeptide which exhibits hepatitis B virus e antigenicity and which is produced by a method as claimed in any of Claims I to 3.

5. A method of preparing a polypeptide exhibiting hepatitis B virus c antigenicity, which comprises cultivating a transformant containing a recombinant DNA comprising a promoter, a translational initiation codon located downstream from the promoter, a DNA fragment which codes for a polypeptide exhibiting hepatitis B virus c antigenicity and which is located downstream from the promoter and a stop codon joined immediately after the DNA fragment, wherein the nucleotide sequence between the SD sequence of the promoter and the translational initiation codon is ATCGGGC.

6. A method according to Claim 5, in which the recombinant DNA is one of the following plasmids,
pTB(4)369 (FERM BP-II53)
pTB(4)369-4
pTB(4)369-32
and
pTB(4)369-4I.

7. A polypeptide which exhibits hepatitis B virus c antigenicity and which is produced by a method as claimed in Claim 4 or 5.

Fig. 1-1



```
         10        20        30        40        50

          ≑         ≑         ≑         ≑         ≑
TTCCACTGCCTTGCACCAAGCTCTGCAGGATCCCAGAGTCAGGGGTCTGT       50
AAGGTGACGGAACGTGGTTCGAGACGTCCTAGGGTCTCAGTCCCCAGACA

          ≑         ≑         ≑         ≑         ≑
ATCTTCCTGCTGGTGGCTCCAGTTCAGGAACAGTAAACCCTGCTCCGAAT      100
TAGAAGGACGACCACCGAGGTCAAGTCCTTGTCATTTGGGACGAGGCTTA

          ≑         ≑         ≑         ≑         ≑
ATTGCCTCTCACATCTCGTCAATCTCCGCGAGGACTGGGGACCCTGTGAC      150
TAACGGAGAGTGTAGAGCAGTTAGAGGCGCTCCTGACCCCTGGGACACTG

          ≑         ≑         ≑         ≑         ≑
GATCATGGAGAACATCACATCAGGATTCCTAGGACCCCTGCTCGTGTTAC      200
CTAGTACCTCTTGTAGTGTAGTCCTAAGGATCCTGGGGACGAGCACAATG

          ≑         ≑         ≑         ≑         ≑
AGGCGGGGTTTTTCTTGTTGACAAGAATCCTCACAATACCGCAGAGTCTA      250
TCCGCCCCAAAAAGAACAACTGTTCTTAGGAGTGTTATGGCGTCTCAGAT

          ≑         ≑         ≑         ≑         ≑
GACTCGTGGTGGACTTCTCTCAATTTTCTAGGGGGATCACCCGTGTGTCT      300
CTGAGCACCACCTGAAGAGAGTTAAAAGATCCCCCTAGTGGGCACACAGA

          ≑         ≑         ≑         ≑         ≑
TGGCCAAAATTCGCAGTCCCCAACCTCCAATCACTCACCAACCTCCTGTC      350
ACCGGTTTTAAGCGTCAGGGGTTGGAGGTTAGTGAGTGGTTGGAGGACAG

          ≑         ≑         ≑         ≑         ≑
CTCCAATTTGTCCTGGTTATCGCTGGATGTGTCTGCGGCGTTTTATCATA      400
GAGGTTAAACAGGACCAATAGCGACCTACACAGACGCCGCAAAATAGTAT

          ≑         ≑         ≑         ≑         ≑
TTCCTCTTCATCCTGCTGCTATGCCTCATCTTCTTATTGGTTCTTCTGGA      450
AAGGAGAAGTAGGACGACGATACGGAGTAGAAGAATAACCAAGAAGACCT

          ≑         ≑         ≑         ≑         ≑
TTATCAAGGTATGTTGCCCGTTTGTCCTCTAATTCCAGGATCAACAACAA      500
AATAGTTCCATACAACGGGCAAACAGGAGATTAAGGTCCTAGTTGTTGTT
```

Fig. 1-2



```
         10         20         30         40         50

          *          *          *          *          *
CCAGTACGGGACCATGCAAAACCTGCACGACTCCTGCTCAAGGCAACTCT      550
GGTCATGCCCTGGTACGTTTTGGACGTGCTGAGGACGAGTTCCGTTGAGA

          *          *          *          *          *
AAGTTTCCCTCATGTTGCTGTACAAAACCTACGGATGGAAATTGCACCTG      600
TTCAAAGGGAGTACAACGACATGTTTTGGATGCCTACCTTTAACGTGGAC

          *          *          *          *          *
TATTCCCATCCCATCGTCCTGGGCTTTCGCAAAATACCTATGGGAGTGGG      650
ATAAGGGTAGGGTAGCAGGACCCGAAAGCGTTTTATGGATACCCTCACCC

          *          *          *          *          *
CCTCAGTCCGTTTCTCTTGGCTCAGTTTACTAGTGCCATTTGTTCAGTGG      700
GGAGTCAGGCAAAGAGAACCGAGTCAAATGATCACGGTAAACAAGTCACC

          *          *          *          *          *
TTCGTAGGGCTTTCCCCCACTGTTTGGCTTTCAGCTATATGGATGATGTG      750
AAGCATCCCGAAAGGGGGTGACAAACCGAAAGTCGATATACCTACTACAC

          *          *          *          *          *
GTATTGGGGGCCAAGTCTGTACAGCATCGTGAGTCCCTTTATACCGCTGT      800
CATAACCCCCGGTTCAGACATGTCGTAGCACTCAGGGAAATATGGCGACA

          *          *          *          *          *
TACCAATTTTCTTTTGTCTCTGGGTATACATTTAAACCCTAACAAAACAA      850
ATGGTTAAAAGAAAACAGAGACCCATATGTAAATTTGGGATTGTTTTGTT

          *          *          *          *          *
AAAGATGGGGTTATTCCCTAAACTTCATGGGCTACATAATTGGAAGTTGG      900
TTTCTACCCCAATAAGGGATTTGAAGTACCCGATGTATTAACCTTCAACC

          *          *          *          *          *
GGAACTTTGCCACAGGATCATATTGTACAAAAGATCAAACACTGTTTTAG      950
CCTTGAAACGGTGTCCTAGTATAACATGTTTTCTAGTTTGTGACAAAATC

          *          *          *          *          *
AAAACTTCCTGTTAACAGGCCTATTGATTGGAAAGTATGTCAAAGAATTG      1000
TTTTGAAGGACAATTGTCCGGATAACTAACCTTTCATACAGTTTCTTAAC
```

Fig. 1-3

```
        10        20        30        40        50

         *         *         *         *         *
TGGGTCTTTTGGGCTTTGCTGCTCCATTTACACAATGTGGATATCCTGCC      1050
ACCCAGAAAACCCGAAACGACGAGGTAAATGTGTTACACCTATAGGACGG

         *         *         *         *         *
TTAATGCCTTTGTATGCATGTATACAAGCTAAACAGGCTTTCACTTTCTC      1100
AATTACGGAAACATACGTACATATGTTCGATTTGTCCGAAAGTGAAAGAG

         *         *         *         *         *
GCCAACTTACAAGGCCTTTCTAAGTAAACAGTACATGAACCTTTACCCCG      1150
CGGTTGAATGTTCCGGAAAGATTCATTTGTCATGTACTTGGAAATGGGGC

         *         *         *         *         *
TTGCTCGGCAACGGCCTGGTCTGTGCCAAGTGTTTGCTGACGCAACCCCC      1200
AACGAGCCGTTGCCGGACCAGACACGGTTCACAAACGACTGCGTTGGGGG

         *         *         *         *         *
ACTGGCTGGGGCTTAGCCATAGGCCATCAGCGCATGCGTGGAACCTTTGT      1250
TGACCGACCCCGAATCGGTATCCGGTAGTCGCGTACGCACCTTGGAAACA

         *         *         *         *         *
GGCTCCTCTGCCGATCCATACTGCGGAACTCCTAGCCGCTTGTTTTGCTC      1300
CCGAGGAGACGGCTAGGTATGACGCCTTGAGGATCGGCGAACAAAACGAG

         *         *         *         *         *
GCAGCCGGTCTGGAGCAAAGCTCATCGGAACTGACAATTCTGTCGTCCTC      1350
CGTCGGCCAGACCTCGTTTCGAGTAGCCTTGACTGTTAAGACAGCAGGAG

         *         *         *         *         *
TCGCGGAAATATACATCATTTCCATGGCTGCTAGGCTGTACTGCCAACTG      1400
AGCGCCTTTATATGTAGTAAAGGTACCGACGATCCGACATGACGGTTGAC

         *         *         *         *         *
GATCCTTCGCGGGACGTCCTTTGTTTACGTCCCGTCGGCGCTGAATCCCG      1450
CTAGGAAGCGCCCTGCAGGAAACAAATGCAGGGCAGCCGCGACTTAGGGC

         *         *         *         *         *
CGGACGACCCCTCTCGGGGCCGCTTGGGACTCTCTCGTCCCCTTCTCCGT      1500
GCCTGCTGGGGAGAGCCCCGGCGAACCCTGAGAGAGCAGGGGAAGAGGCA
```

Fig. 1-4



10 20 30 40 50

≑ ≑ ≑ ≑ ≑
CTGCCGTTCCAGCCGACCACGGGGCGCACCTCTCTTTACGCGGTCTCCCC 1550
GACGGCAAGGTCGGCTGGTGCCCCGCGTGGAGAGAAATGCGCCAGAGGGG

≑ ≑ ≑ ≑ ≑
GTCTGTGCCTTCTCATCTGCCGGTCCGTGTGCACTTCGCTTCACCTCTGC 1600
CAGACACGGAAGAGTAGACGGCCAGGCACACGTGAAGCGAAGTGGAGACG

≑ ≑ ≑ ≑ ≑
ACGTTGCATGGCGACCACCGTGAACGCCCATCAGATCCTGCCCAAGGTCT 1650
TGCAACGTACCGCTGGTGGCACTTGCGGGTAGTCTAGGACGGGTTCCAGA

≑ ≑ ≑ ≑ ≑
TACATAAGAGGACTCTTGGACTCCCAGCAATGTCAACGACCGACCTTGAG 1700
ATGTATTCTCCTGAGAACCTGAGGGTCGTTACAGTTGCTGGCTGGAACTC

≑ ≑ ≑ ≑ ≑
GCCTACTTCAAAGACTGTGTGTTTAAGGACTGGGAGGAGTTGGGGGAGGA 1750
CGGATGAAGTTTCTGACACACAAATTCCTGACCCTCCTCAACCCCCTCCT

≑ ≑ ≑ ≑ ≑
GATTAGGTTAATGATCTTTGTATTAGGAGGCTGTAGGCATAAATTGGTCT 1800
CTAATCCAATTACTAGAAACATAATCCTCCGACATCCGTATTTAACCAGA

≑ ≑ ≑ ≑ ≑
GCGCACCAGCACCATGCAACTTTTTCACCTCTGCCTAATCATCTCTTGTA 1850
CGCGTGGTCGTGGTACGTTGAAAAAGTGGAGACGGATTAGTAGAGAACAT

≑ ≑ ≑ ≑ ≑
CATGTCCCACTGTTCAAGCCTCCAAGCTGTGCCTTGGGTGGCTTTGGGGC 1900
GTACAGGGTGACAAGTTCGGAGGTTCGACACGGAACCCACCGAAACCCCG

≑ ≑ ≑ ≑ ≑
ATGGACATTGACCCTTATAAAGAATTTGGAGCTACTGTGGAGTTACTCTC 1950
TACCTGTAACTGGGAATATTTCTTAAACCTCGATGACACCTCAATGAGAG

≑ ≑ ≑ ≑ ≑
GTTTTTGCCTTCTGACTTCTTTCCTTCCGTACGAGATCTCCTAGACACCG 2000
CAAAAACGGAAGACTGAAGAAAGGAAGGCATGCTCTAGAGGATCTGTGGC

Fig. 1-5



```
      10        20        30        40        50

       *         *         *         *         *
CCTCAGCTCTGTATCGAGAAGCCTTAGAGTCTCCTGAGCATTGCTCACCT     2050
GGAGTCGAGACATAGCTCTTCGGAATCTCAGAGGACTCGTAACGAGTGGA

       *         *         *         *         *
CACCATACTGCACTCAGGCAAGCCATTCTCTGCTGGGGGGAATTGATGAC     2100
GTGGTATGACGTGAGTCCGTTCGGTAAGAGACGACCCCCCTTAACTACTG

       *         *         *         *         *
TCTAGCTACCTGGGTGGGTAATAATTTGCAAGATCCAGCATCCAGAGATC     2150
AGATCGATGGACCCACCCATTATTAAACGTTCTAGGTCGTAGGTCTCTAG

       *         *         *         *         *
TAGTAGTCAATTATGTTAATACTAACATGGGTTTAAAGATCAGGCAACTA     2200
ATCATCAGTTAATACAATTATGATTGTACCCAAATTTCTAGTCCGTTGAT

       *         *         *         *         *
TTGTGGTTTCATATATCTTGCCTTACTTTTGGAAGAGAGACTGTACTTGA     2250
AACACCAAAGTATATAGAACGGAATGAAAACCTTCTCTCTGACATGAACT

       *         *         *         *         *
ATATTTGGTCTCTTTCGGAGTGTGGATTCGCACTCCTCCAGCCTATAGAC     2300
TATAAACCAGAGAAAGCCTCACACCTAAGCGTGAGGAGGTCGGATATCTG

       *         *         *         *         *
CACCAAATGCCCCTATCTTATCAACACTTCCGGAAACTACTGTTGTTAGA     2350
GTGGTTTACGGGGATAGAATAGTTGTGAAGGCCTTTGATGACAACAATCT

       *         *         *         *         *
CGACGGGACCGAGGCAGGTCCCCTAGAAGAAGAACTCCCTCGCCTCGCAG     2400
GCTGCCCTGGCTCCGTCCAGGGGATCTTCTTCTTGAGGGAGCGGAGCGTC

       *         *         *         *         *
ACGCAGATCTCAATCGCCGCGTCGCAGAAGATCTCAATCTCGGGAATCTC     2450
TGCGTCTAGAGTTAGCGGCGCAGCGTCTTCTAGAGTTAGAGCCCTTAGAG

       *         *         *         *         *
AATGTTAGTATTCCTTGGACTCATAAGGTCGGAAACTTTACGGGGCTTTA     2500
TTACAATCATAAGGAACCTGAGTATTCCAGCCTTTGAAATGCCCCGAAAT
```

Fig. 1-6 

```
         10        20        30        40        50
          ≑         ≑         ≑         ≑         ≑
TTCCTCTACAGTACCTATCTTTAATCCTGAATGGCAAACTCCTTCCTTTC      2550
AAGGAGATGTCATGGATAGAAATTAGGACTTACCGTTTGAGGAAGGAAAG

          ≑         ≑         ≑         ≑         ≑
CTAAGATTCATTTACAAGAGGACATTATTAATAGGTGTCAACAATTTGTG      2600
GATTCTAAGTAAATGTTCTCCTGTAATAATTATCCACAGTTGTTAAACAC

          ≑         ≑         ≑         ≑         ≑
GGCCCTCTCACTGTAAATGAAAAGAGAAGATTGAAATTAATTATGCCTGC      2650
CCGGGAGAGTGACATTTACTTTTCTCTTCTAACTTTAATTAATACGGACG

          ≑         ≑         ≑         ≑         ≑
TAGATTCTATCCTACCCACACTAAATATTTGCCCTTAGACAAAGGAATTA      2700
ATCTAAGATAGGATGGGTGTGATTTATAAACGGGAATCTGTTTCCTTAAT

          ≑         ≑         ≑         ≑         ≑
AACCTTATTATCCAGATCAGGTAGTTAATCATTACTTCCAAACCAGACAT      2750
TTGGAATAATAGGTCTAGTCCATCAATTAGTAATGAAGGTTTGGTCTGTA

          ≑         ≑         ≑         ≑         ≑
TATTTACATACTCTTTGGAAGGCTGGTATTCTATATAAGAGGGAAACCAC      2800
ATAAATGTATGAGAAACCTTCCGACCATAAGATATATTCTCCCTTTGGTG

          ≑         ≑         ≑         ≑         ≑
ACGTAGCGCATCATTTTGCGGGTCACCATATTCTTGGGAACAAGAGCTAC      2850
TGCATCGCGTAGTAAAACGCCCAGTGGTATAAGAACCCTTGTTCTCGATG

          ≑         ≑         ≑         ≑         ≑
AGCATTCGCAAAGGCATGGGGACGAATCTTTCTGTTCCCAACCCTCTGGG      2900
TCGTAAGCGTTTCCGTACCCCTGCTTAGAAAGACAAGGGTTGGGAGACCC

          ≑         ≑         ≑         ≑         ≑
ATTCCTTCCCGATCATCAGTTGGACCCTGCATTCGGAGCCAACTCAACAA      2950
TAAGGAAGGGCTAGTAGTCAACCTGGGACGTAAGCCTCGGTTGAGTTGTT

          ≑         ≑         ≑         ≑         ≑
ATCCAGATTGGGACTTCAACCCCATCAAGGACCACTGGCCAGCAGCCAAC      3000
TAGGTCTAACCCTGAAGTTGGGGTAGTTCCTGGTGACCGGTCGTCGGTTG
```

Fig. 1-7

```
        10        20        30        40        50

         ≑         ≑         ≑         ≑         ≑
CAGGTAGGAGTGGGAGCATTCGGGCCAGGGCTCACCCCTCCACACGGCGG    3050
GTCCATCCTCACCCTCGTAAGCCCGGTCCCGAGTGGGGAGGTGTGCCGCC

         ≑         ≑         ≑         ≑         ≑
TATTTTGGGGTGGAGCCCTCAGGCTCAGGGCATATTGACCACAGTGTCAA    3100
ATAAAACCCCACCTCGGGAGTCCGAGTCCCGTATAACTGGTGTCACAGTT

         ≑         ≑         ≑         ≑         ≑
CAATTCCTCCTCCTGCCTCCACCAATCGGCAGTCAGGAAGGCAGCCTACT    3150
GTTAAGGAGGAGGACGGAGGTGGTTAGCCGTCAGTCCTTCCGTCGGATGA

         ≑         ≑         ≑         ≑         ≑
CCCATCTCTCCACCTCTAAGAGACAGTCATCCTCAGGCCATGCAGTGGAA    3200
GGGTAGAGAGGTGGAGATTCTCTGTCAGTAGGAGTCCGGTACGTCACCTT
```

Fig. 2

Met Asp Ile Asp Pro Tyr Lys Glu Phe Gly Ala Thr Val Glu Leu
Leu Ser Phe Leu Pro Ser Asp Phe Phe Pro Ser Val Arg Asp Leu
Leu Asp Thr Ala Ser Ala Leu Tyr Arg Glu Ala Leu Glu Ser Pro
Glu His Cys Ser Pro His His Thr Ala Leu Arg Gln Ala Ile Leu
Cys Trp Gly Glu Leu Met Thr Leu Ala Thr Trp Val Gly Asn Asn
Leu Gln Asp Pro Ala Ser Arg Asp Leu Val Val Asn Tyr Val Asn
Thr Asn Met Gly Leu Lys Ile Arg Gln Leu Leu Trp Phe His Ile
Ser Cys Leu Thr Phe Gly Arg Glu Thr Val Leu Glu Tyr Leu Val
Ser Phe Gly Val Trp Ile Arg Thr Pro Pro Ala Tyr Arg Pro Pro
Asn Ala Pro Ile Leu Ser Thr Leu Pro Glu Thr Thr Val Val Arg
Arg Arg Asp Arg Gly Arg Ser Pro Arg Arg Arg Thr Pro Ser Pro
Arg Arg Arg Arg Ser Gln Ser Pro Arg Arg Arg Arg Ser Gln Ser
Arg Glu Ser Gln Cys -

Fig. 3



```
         10        20        30        40        50
          ≑         ≑         ≑         ≑         ≑
ATGGACATTGACCCTTATAAAGAATTTGGAGCTACTGTGGAGTTACTCTC    50

          ≑         ≑         ≑         ≑         ≑
GTTTTTGCCTTCTGACTTCTTTCCTTCCGTACGAGATCTCCTAGACACCG   100

          ≑         ≑         ≑         ≑         ≑
CCTCAGCTCTGTATCGAGAAGCCTTAGAGTCTCCTGAGCATTGCTCACCT   150

          ≑         ≑         ≑         ≑         ≑
CACCATACTGCACTCAGGCAAGCCATTCTCTGCTGGGGGGAATTGATGAC   200

          ≑         ≑         ≑         ≑         ≑
TCTAGCTACCTGGGTGGGTAATAATTTGCAAGATCCAGCATCCAGAGATC   250

          ≑         ≑         ≑         ≑         ≑
TAGTAGTCAATTATGTTAATACTAACATGGGTTTAAAGATCAGGCAACTA   300

          ≑         ≑         ≑         ≑         ≑
TTGTGGTTTCATATATCTTGCCTTACTTTTGGAAGAGAGACTGTACTTGA   350

          ≑         ≑         ≑         ≑         ≑
ATATTTGGTCTCTTTCGGAGTGTGGATTCGCACTCCTCCAGCCTATAGAC   400

          ≑         ≑         ≑         ≑         ≑
CACCAAATGCCCCTATCTTATCAACACTTCCGGAAACTACTGTTGTTAGA   450

          ≑         ≑         ≑         ≑         ≑
CGACGGGACCGAGGCAGGTCCCCTAGAAGAAGAACTCCCTCGCCTCGCAG   500

          ≑         ≑         ≑         ≑         ≑
ACGCAGATCTCAATCGCCGCGTCGCAGAAGATCTCAATCTCGGGAATCTC   550

          ≑         ≑         ≑         ≑         ≑
AATGTTAG
```

Fig. 4

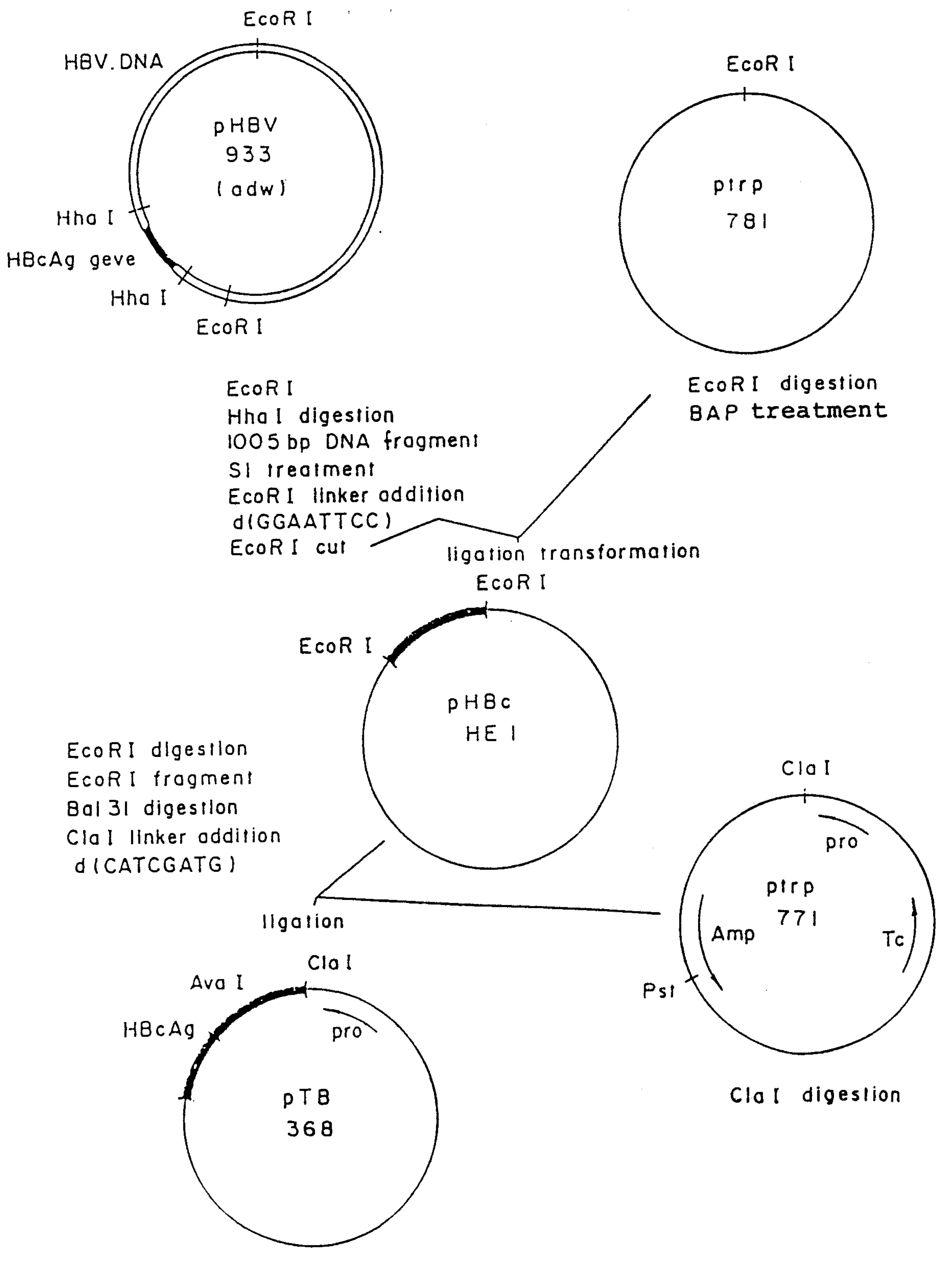
EcoR I
HBV. DNA
pHBV
933
(adw)
Hha I
HBcAg geve
Hha I
EcoR I
EcoR I
ptrp
781
EcoR I
Hha I digestion
1005 bp DNA fragment
S1 treatment
EcoR I linker addition
d(GGAATTCC)
EcoR I cut
EcoR I digestion
BAP treatment
ligation transformation
EcoR I
EcoR I
pHBc
HE I
EcoR I digestion
EcoR I fragment
Bal 31 digestion
Cla I linker addition
d(CATCGATG)
Cla I
pro
ptrp
771
Amp
Tc
Pst
ligation
Cla I
Ava I
HBcAg
pro
pTB
368
Cla I digestion

Fig. 5

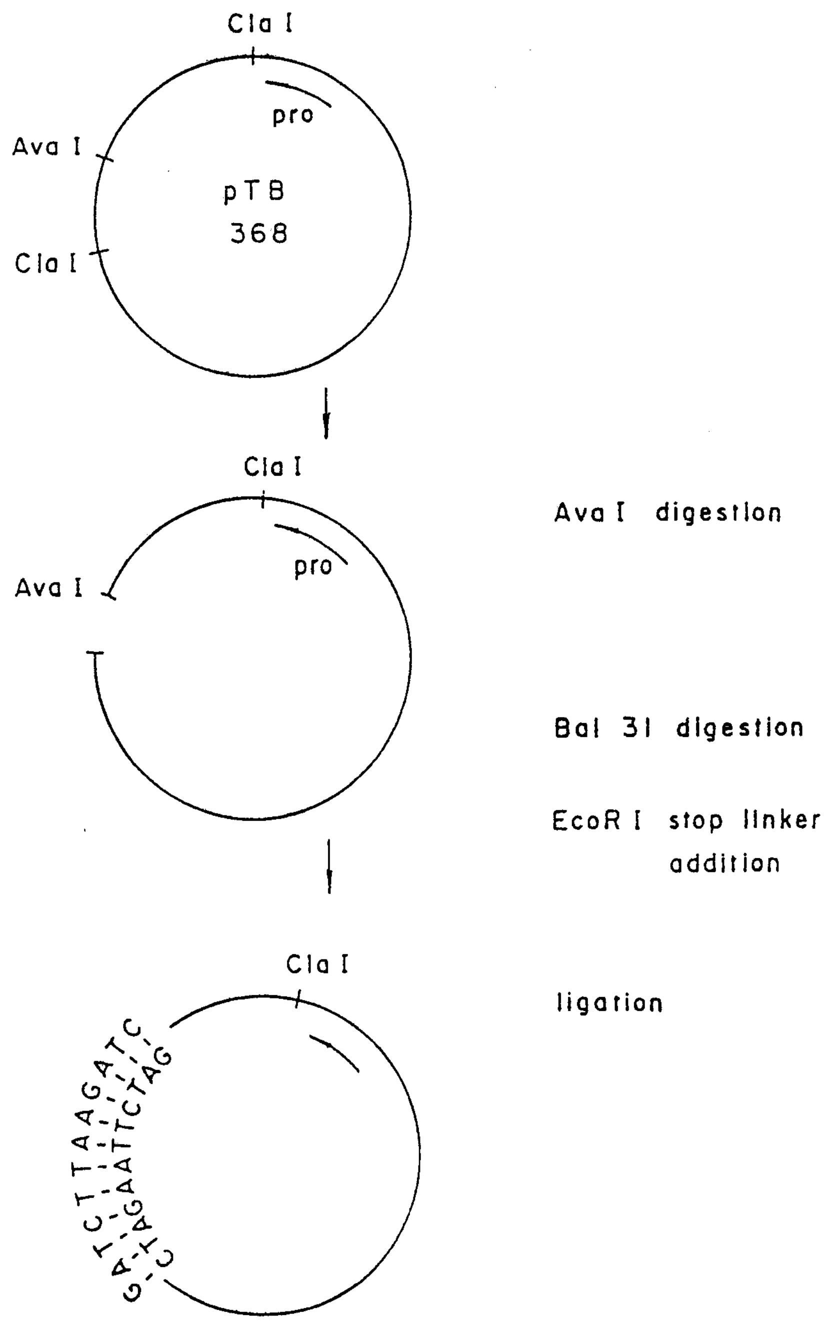
Cla I
pro
Ava I
pTB
368
Cla I
Cla I
pro
Ava I
Ava I digestion
Bal 31 digestion
EcoR I stop linker
addition
Cla I
ligation
GATCTTAAGATC
CTAGAATTCTAG

Fig. 6

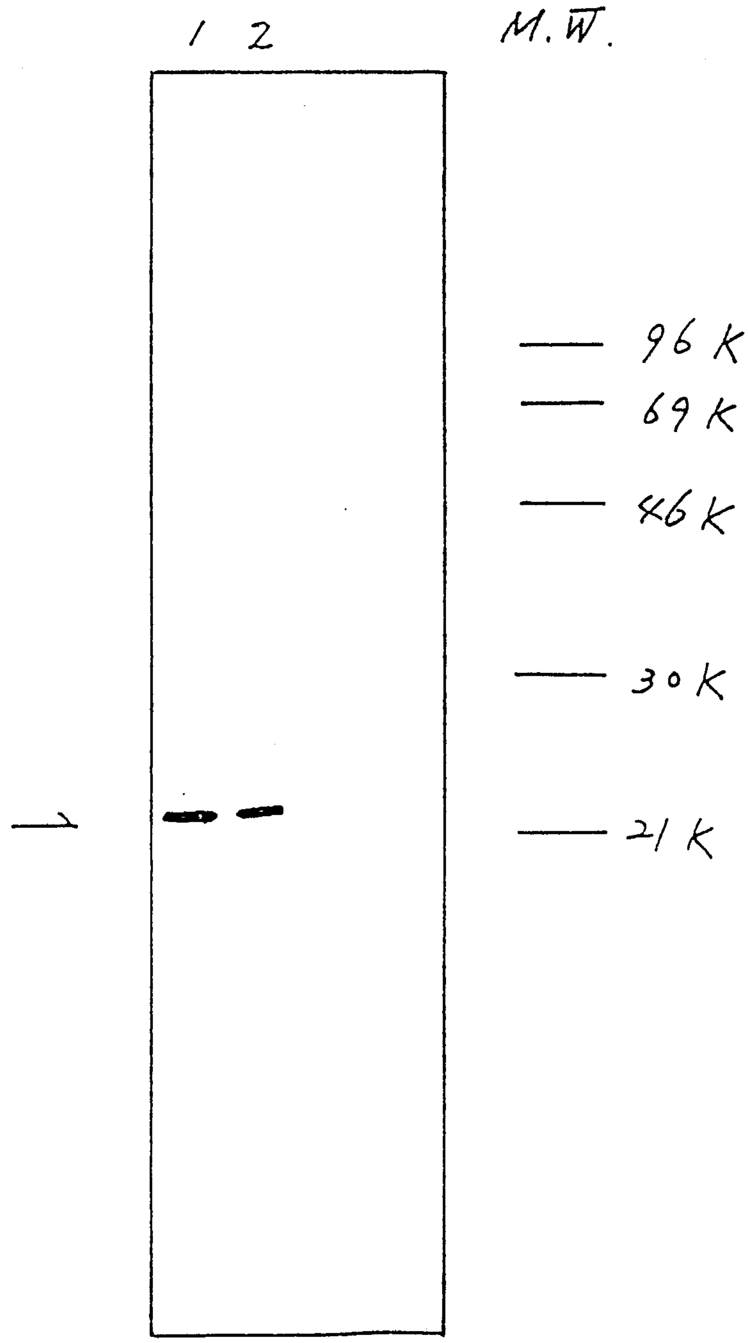

0218474

Fig. 7

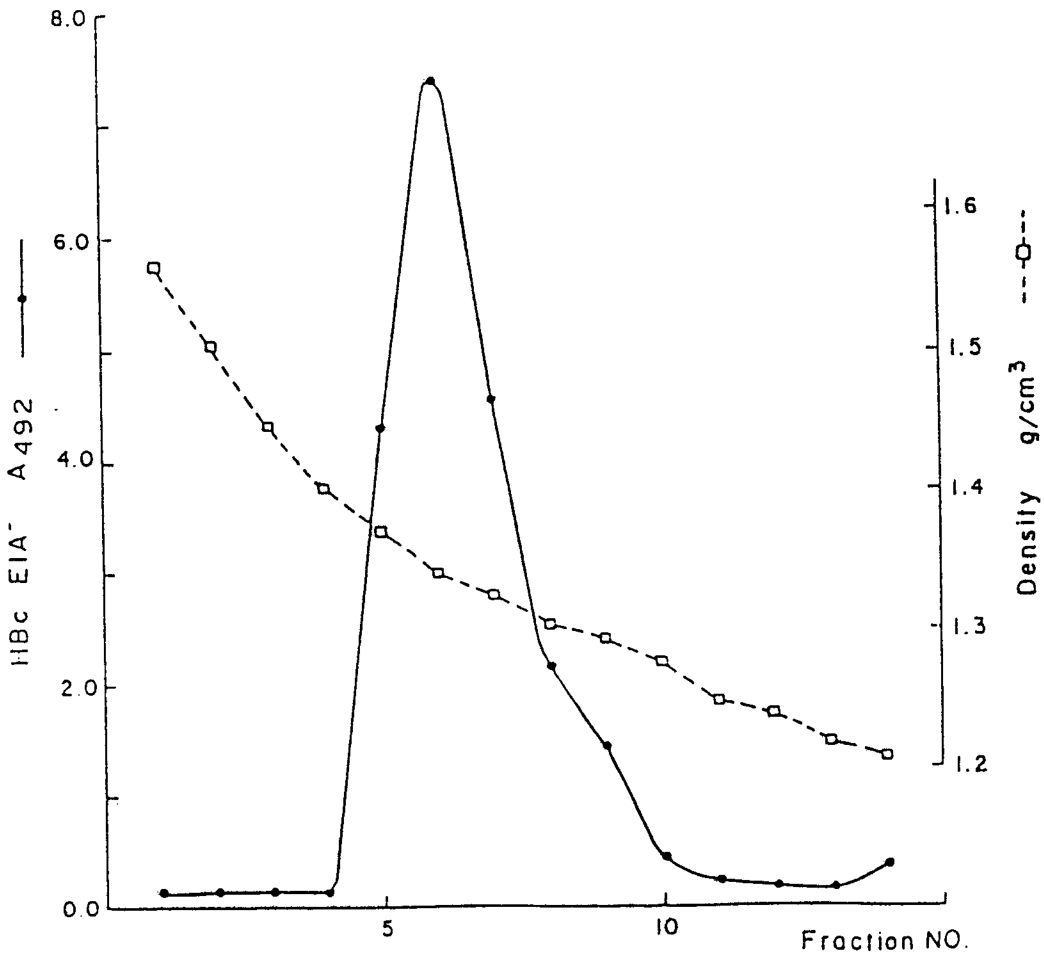

Fig. 8

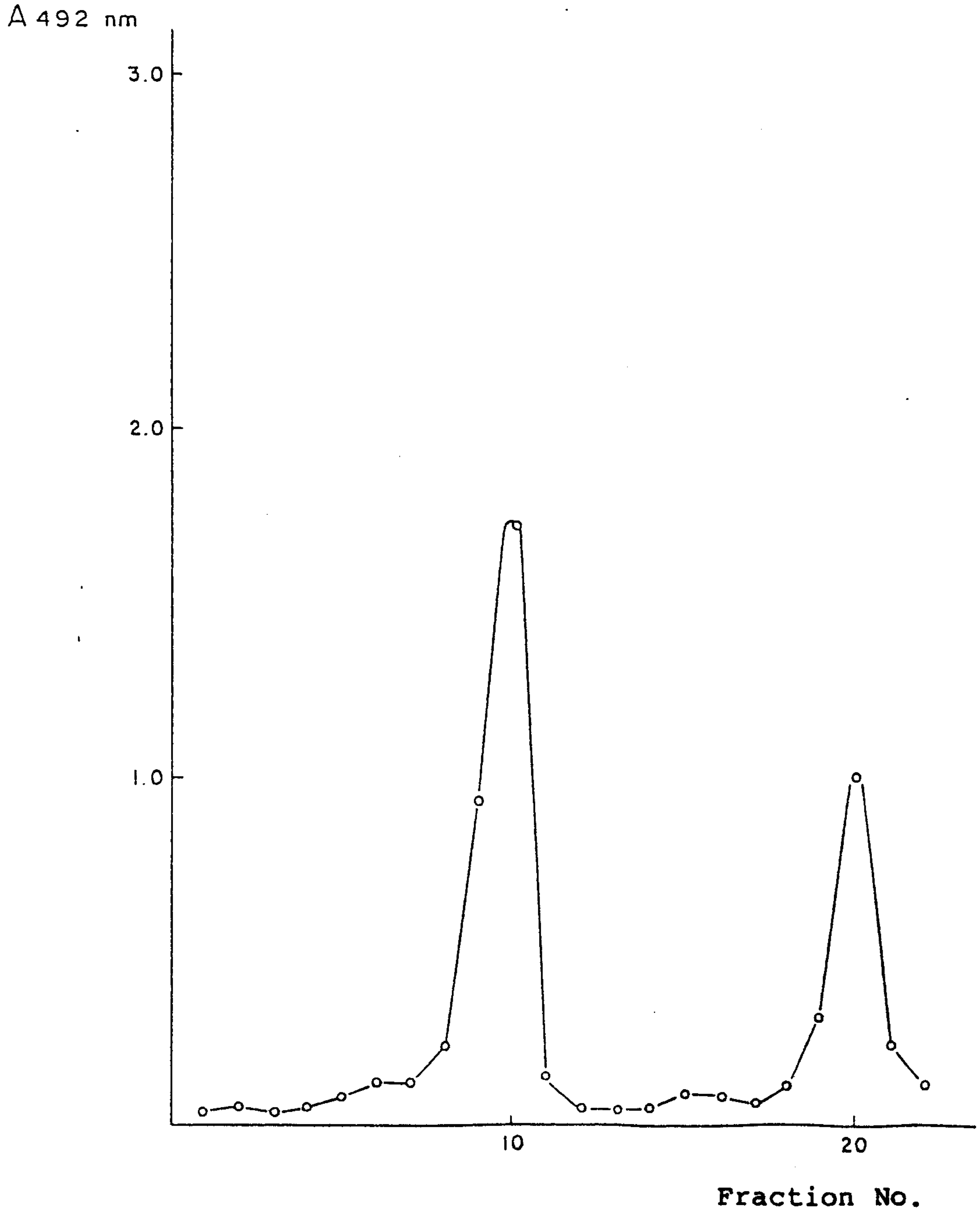

Fig. 9

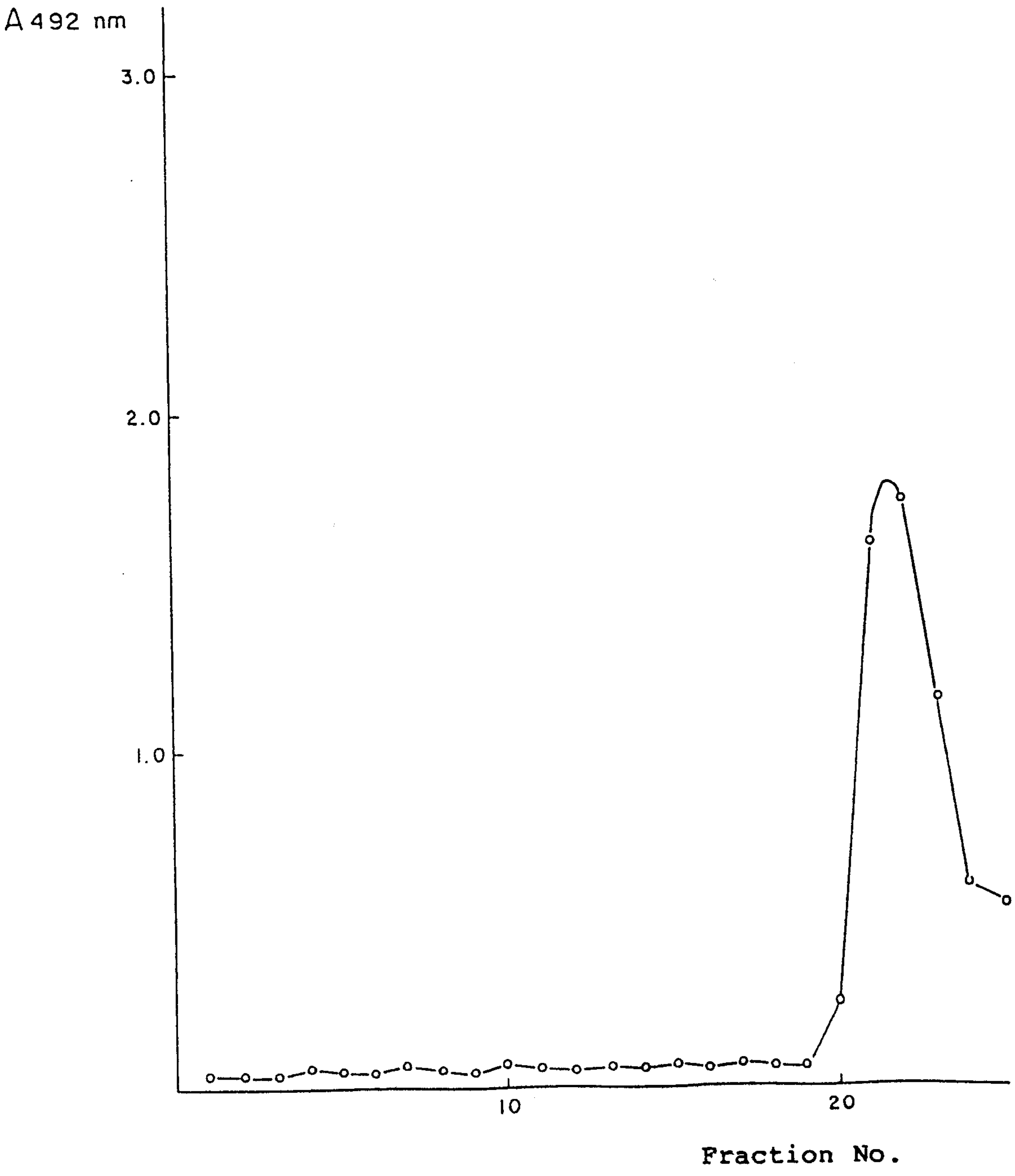

Fig. 10

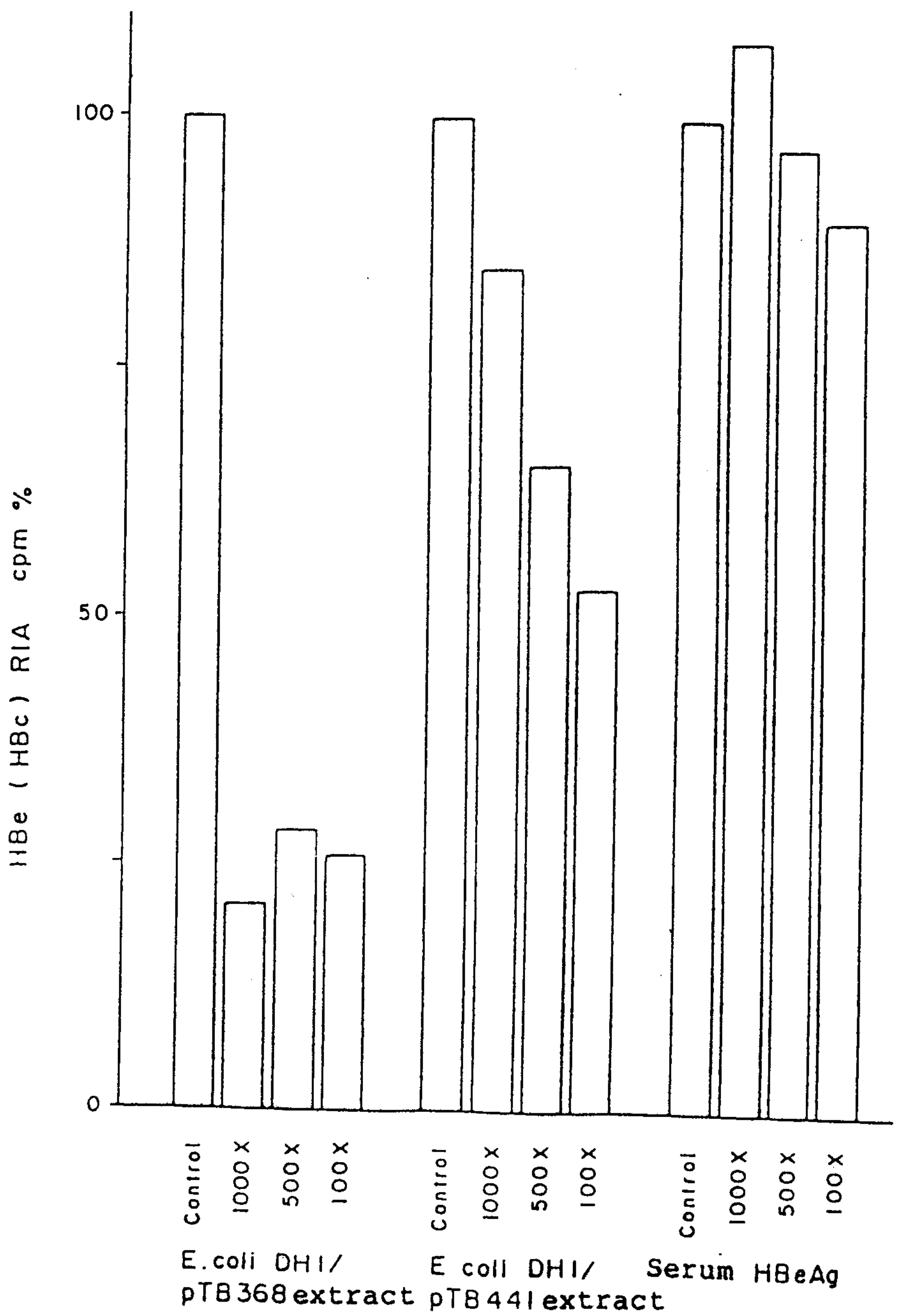
HBe ( HBc ) RIA cpm %
100
50
0
Control
1000X
500X
100X
Control
1000X
500X
100X
Control
1000X
500X
100X
E.coli DH1/
pTB368 extract
E coli DH1/
pTB441 extract
Serum HBeAg

Fig. 11

Construction of the plasmid pTB(4) 369-4

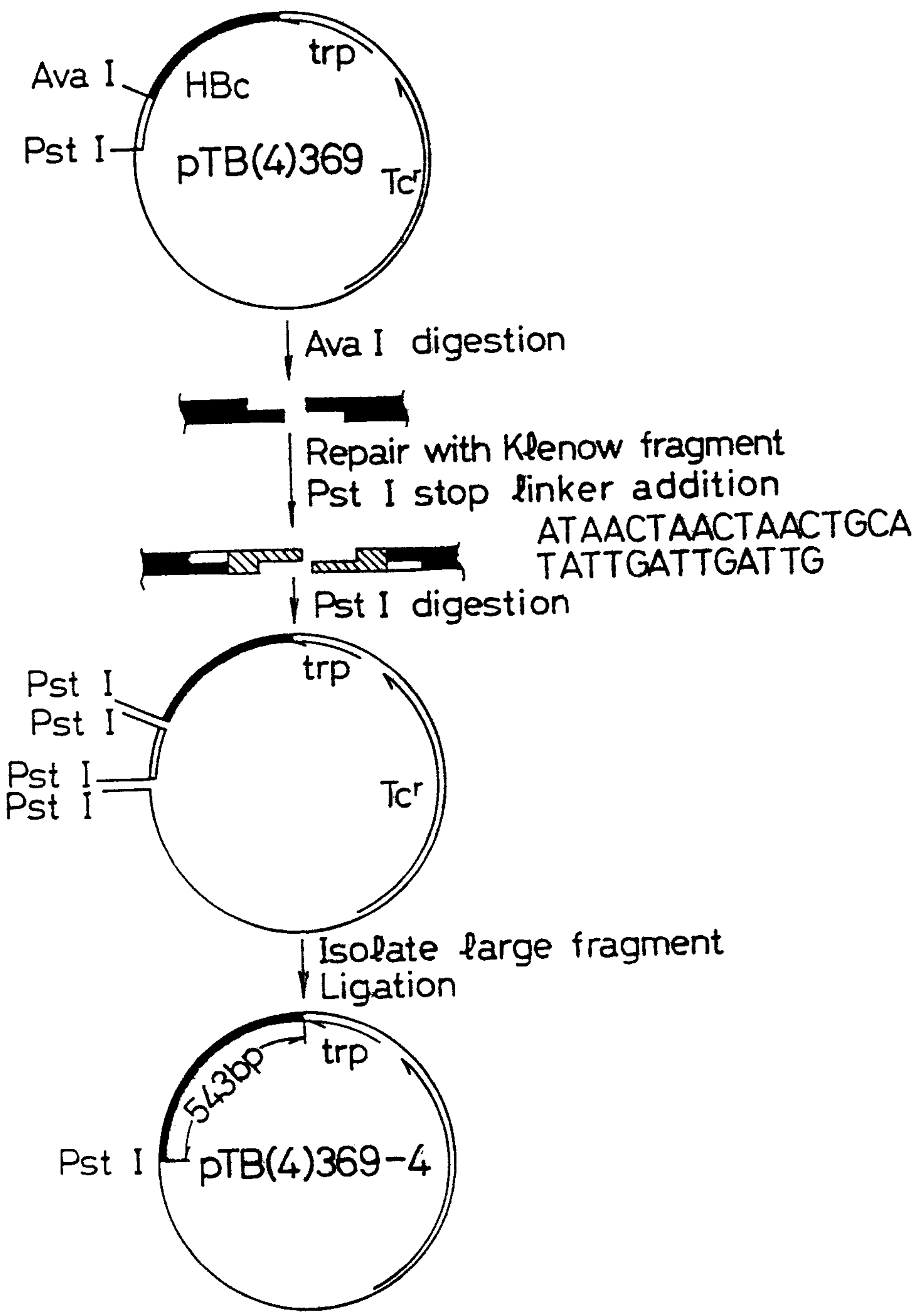

Fig. 12

Construction of the plasmids pTB(4) 369-32 and pTB(4) 369-41

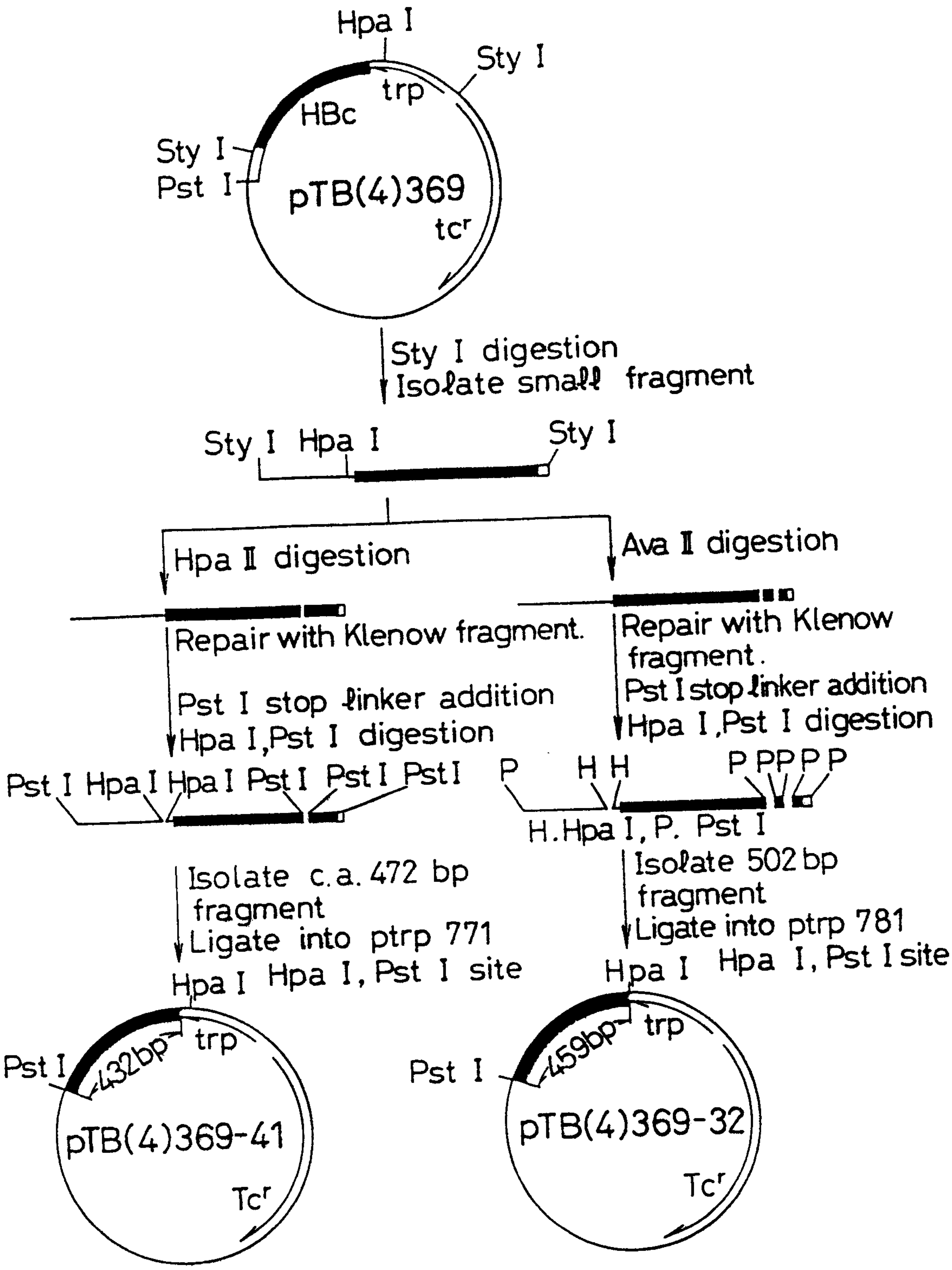

Fig. 13

Construction of the plasmid pTB(4) 441-1

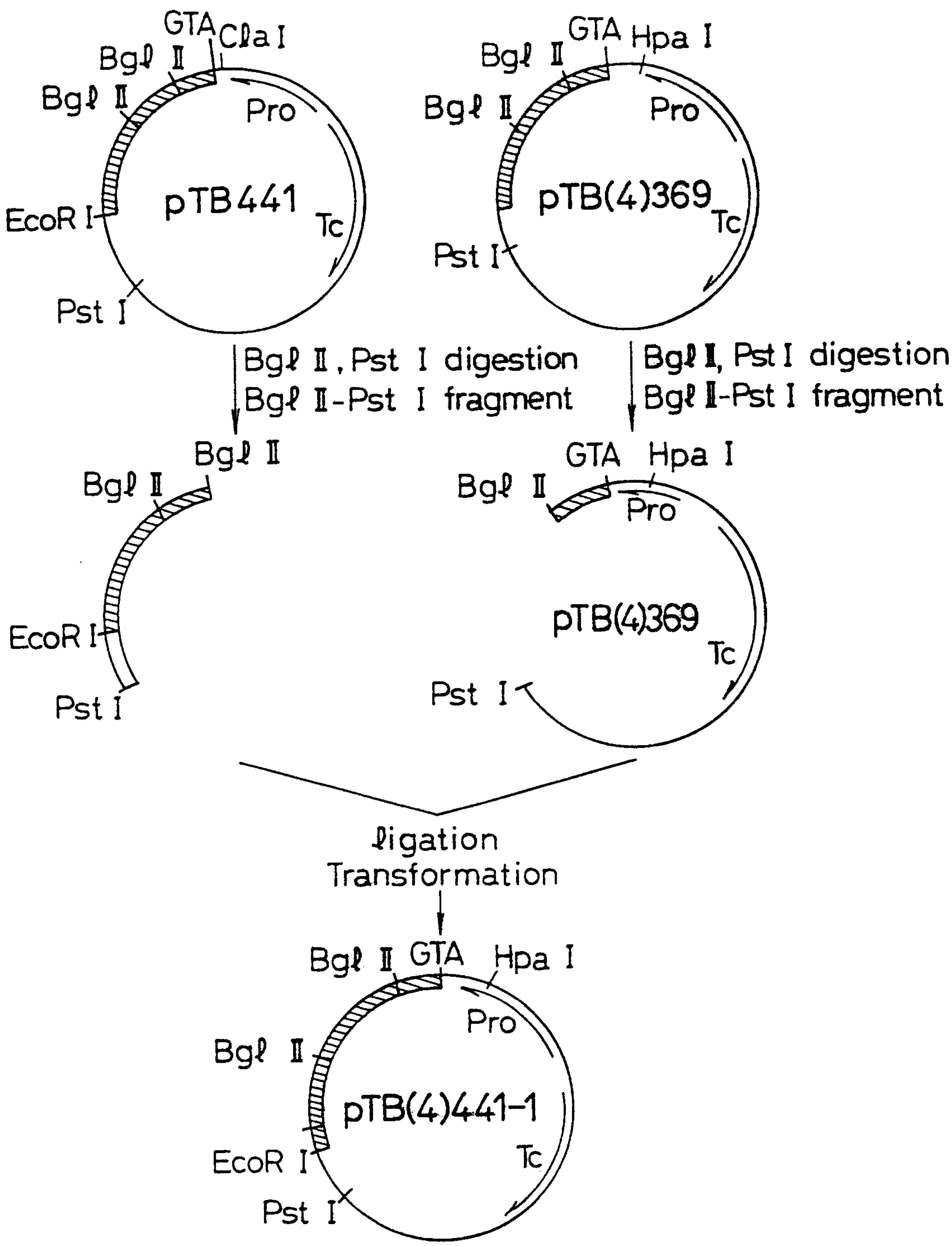

Fig. 14

Construction of the plasmid pTB(4) 441-8

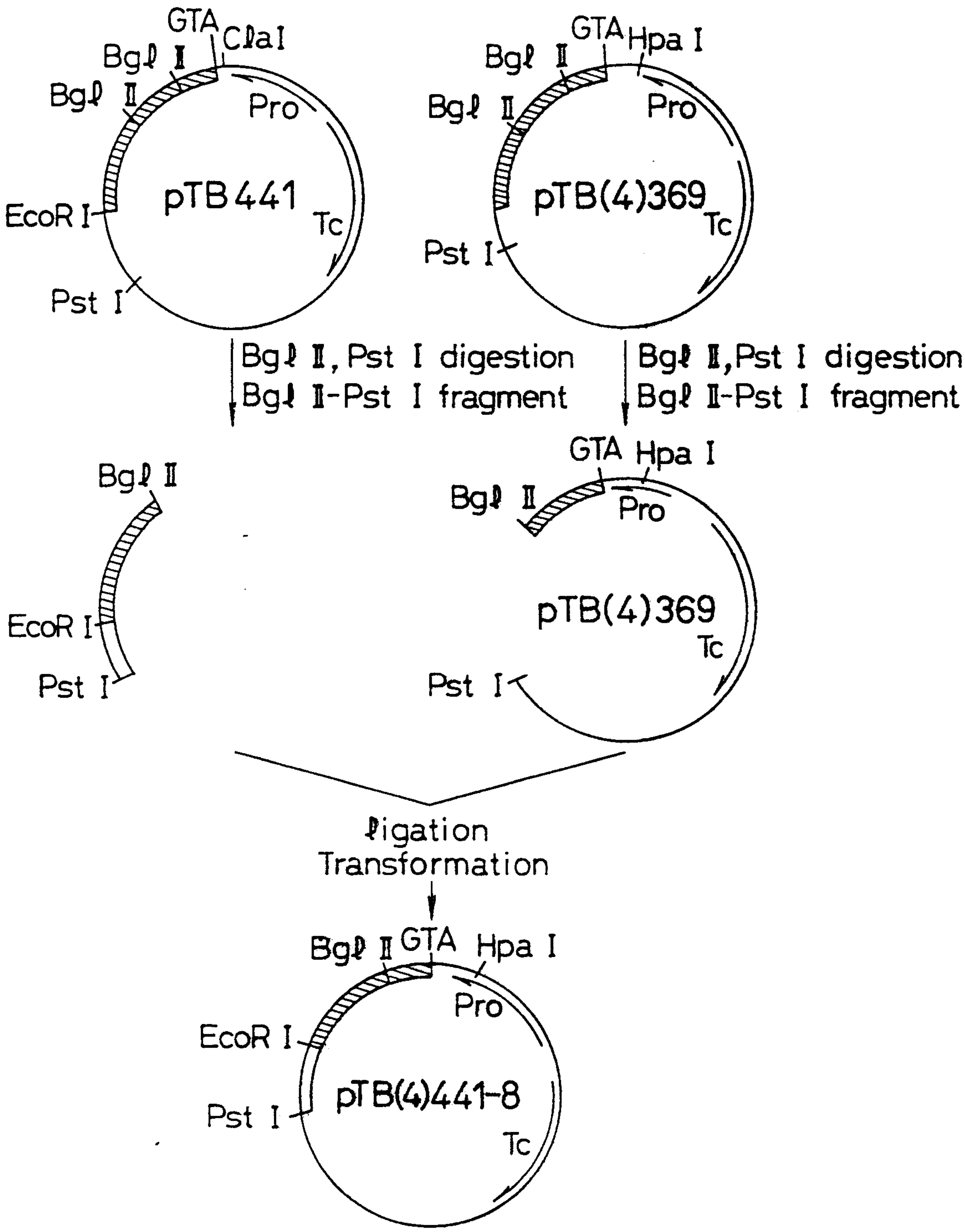

Fig. 15

Construction of the plasmid pTB(4) 552-8

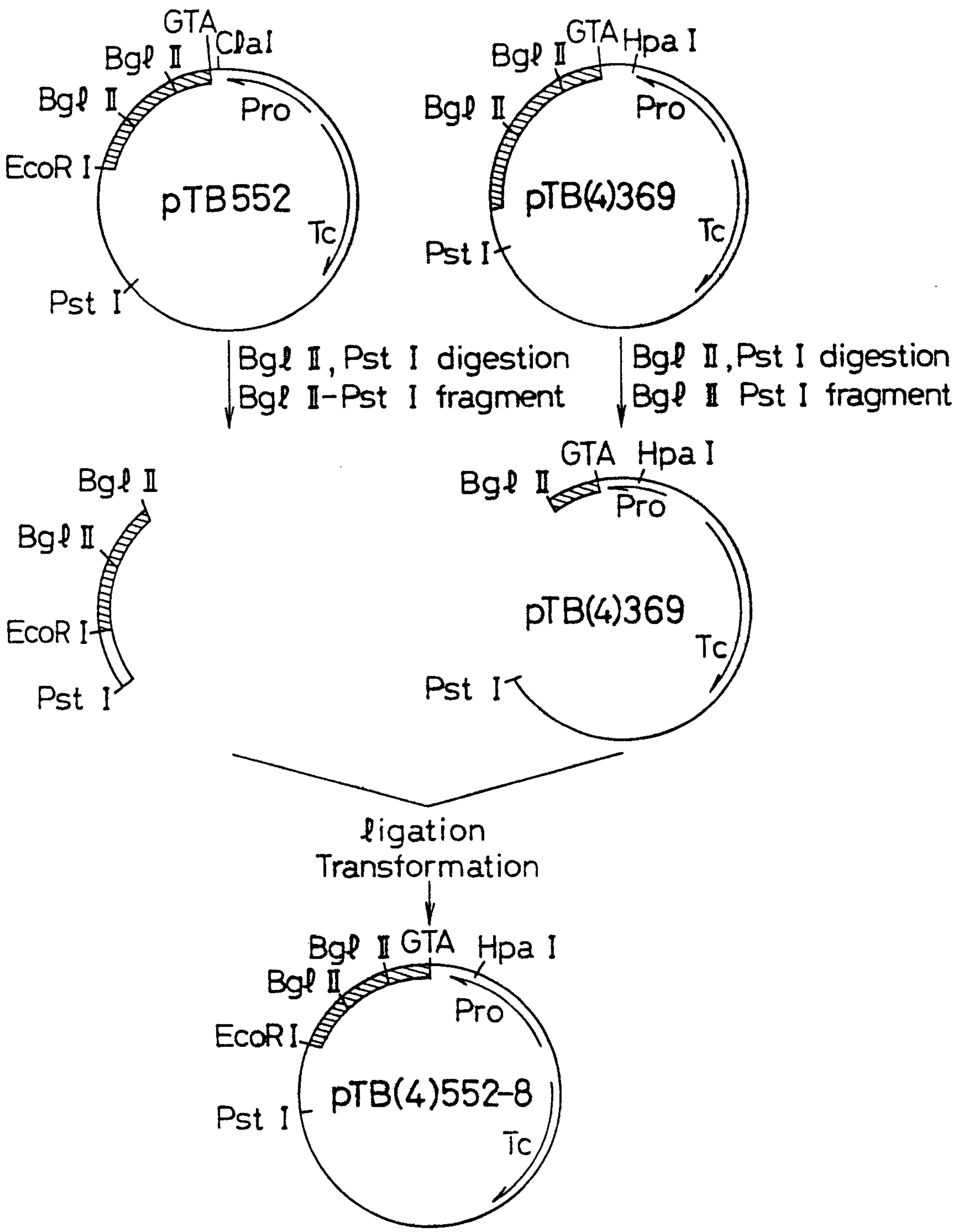

Fig. 16

Western Blotting

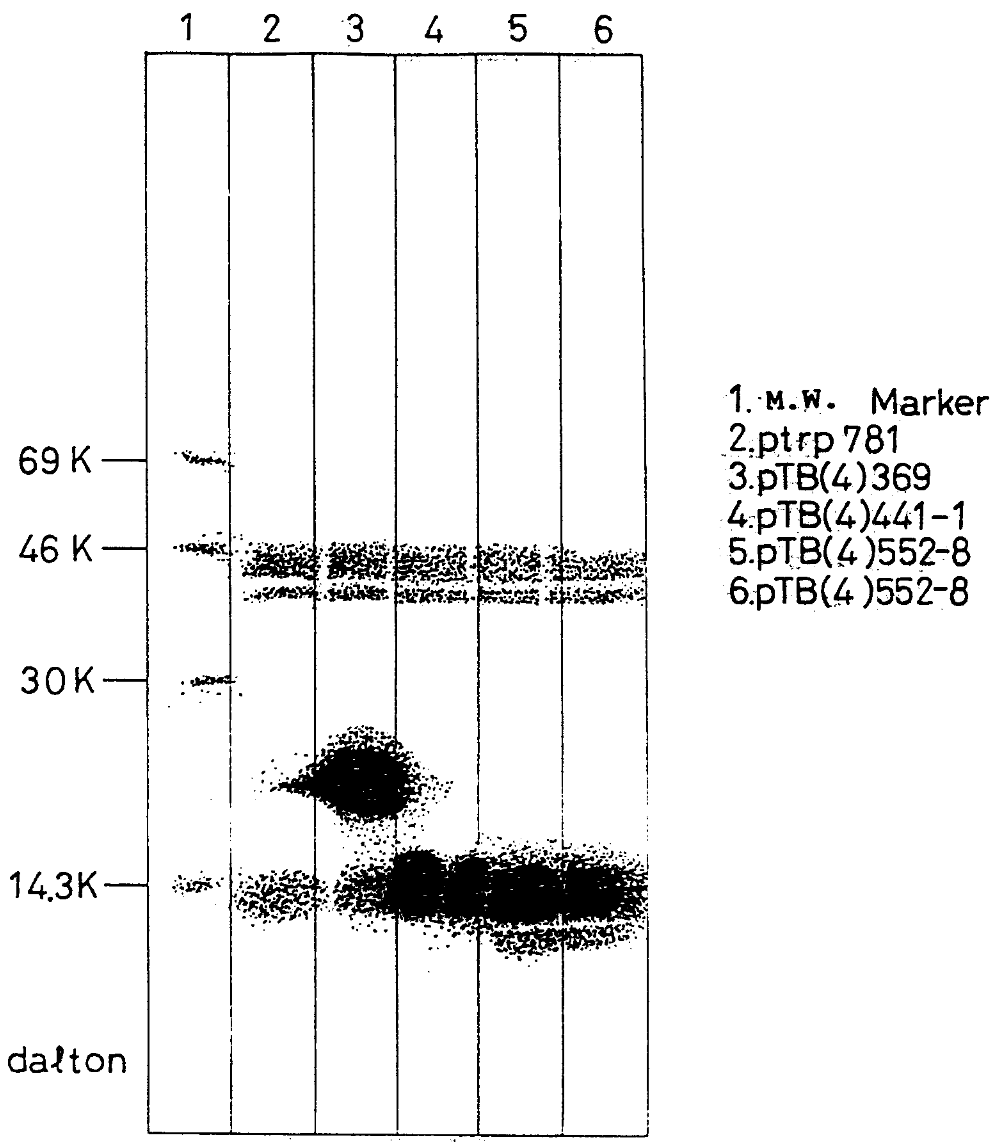

Fig. 17

Fluorography of polyacrylamide gel electrophoresis of pTB(4)552-8 immunoprecipitates($^{14}C$ labelled)

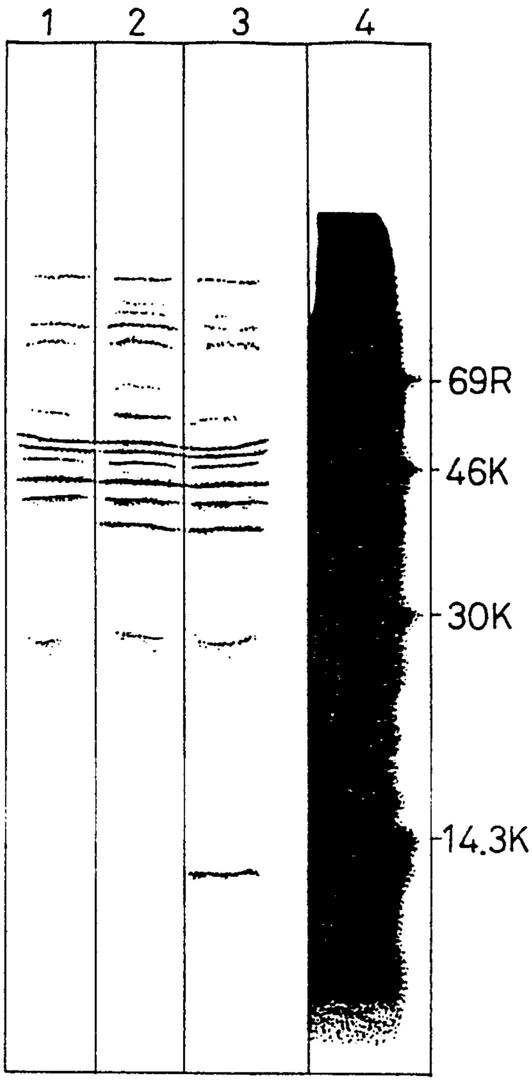


1. Normal human serum + $^{14}C$ pTB(4) 552-8 extract

2. Anti-HBe anti serum (+10μl) + $^{14}C$ pTB(4)552-8 extract

3. Anti-HBe anti serum (+30 μl) $^{14}C$ pTB(4) 552-8 extract

4. M.W. marker